# EUROPEAN PATENT SPECIFICATION

(11) **EP 0 389 421 B1**
(45) Date of publication and mention of the grant of the patent: **27.04.1994**
(21) Application number: 90810189.2
(22) Date of filing: 13.03.1990
(51) Int. Cl.: C07D 211/94, C08K 5/3435

(54) **Poly(1-hydrocarbyloxy-2,2,6,6-tetraalkylpiperidine) light stabilizers**
Poly(1-hydrocarbyloxy-2,2,6,6-tetraalkylpiperidin)- Lichtstabilisatoren
Poly(1-hydrocarbyloxy-2,2,6,6-tétraalkylpipéridines), stabilisateurs contre la lumière

(30) Priority: 21.03.1989 US 326703; 21.03.1989 US 326704
(43) Date of publication of application: 26.09.1990
(73) Proprietor: CIBA-GEIGY AG, 4002 Basel (CH)
(72) Inventor: Galbo, James P., Hartsdale, N.Y. 10530 (US)

(56) References cited:
- EP-A- 0 224 720
- EP-A- 0 290 393
- EP-A- 0 300 160
- FR-A- 2 405 247
- US-A- 4 191 829

## Description

The instant invention pertains to polysubstituted and oligomeric N-hydrocarbyloxy hindered amines and polymer compositions containing said amines which are stabilized against the deleterious effects of actinic light.

Monomeric N-hydrocarbyloxy hindered amines are described in EP-A-3O9 4O2.

Copolymers of bis(N-oxylpiperidines) with p-xylylene have been reported in Japanese patent application 79/42000; T. Fujita et al, J. Poly Sci, Poly Chem. Ed, 18, 549 (1980) and 20, 1639 (1982); and J. Poly Sci, Polym Letter Ed, 16, 515 (1978); 17, 353 (1979) and 19, 609 (1981).

The instant polysubstituted and oligomeric N-hydrocarbyloxy derivatives prepared from hindered amines and saturated or unsaturated aliphatic hydrocarbons are not described or suggested in the prior art.

The instant invention pertains to poly(1-hydrocarbyloxy-2,2,6,6-tetraalkylpiperidine) compounds of formula I or II 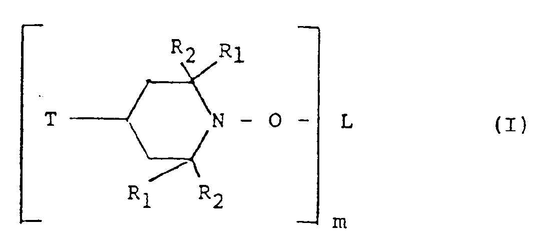 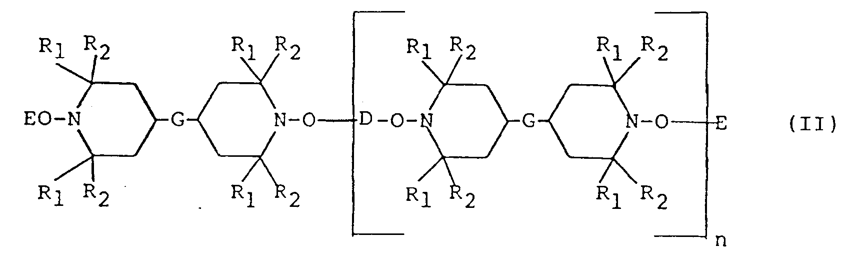
where
m is 2 to 10, n is 1 to 5,
R₁ and R₂ are independently alkyl of 1 to 4 carbon atoms, or R₁ and R₂ together are pentamethylene,
L is an m-valent radical of an alkane of 1 to 18 carbon atoms or of an alkene of up to 18 carbon atoms, an m-valent radical of a cycloalkane or cycloalkene of 5 to 12 carbon atoms, an m-valent radical of a saturated or unsaturated bicyclic or tricyclic hydrocarbon of 7 to 12 carbon atoms or an m-valent radical of an aryl, alkyl substituted aryl or aralkyl hydrocarbon of 6 to 15 carbon atoms, with the proviso that the N-O groups are not necessarily attached to the same carbon atom in L,
T is an organic moiety selected from the group consisting of R₃-COO-, R₄-OCO-, R₃-CONR₅-, 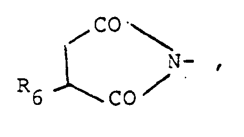
R₄NHCOCONH-, 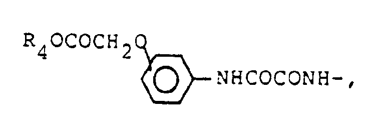
R₄OCO(CH₂)_{q}NH-, R₄OCO-(CH₂)ₚCOO-, 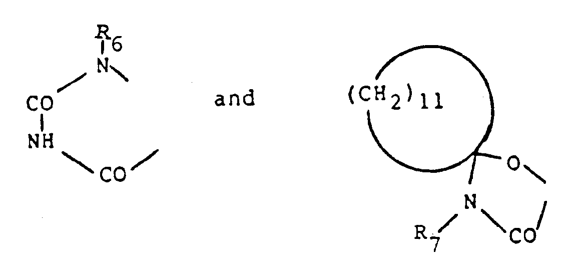
where
R₃ is a radical of an aliphatic, cycloaliphatic, unsaturated aliphatic or aromatic carboxylic acid without the carboxy group,
R₄ is a radical of an aliphatic, cycloaliphatic or unsaturated aliphatic alcohol without the hydroxy group,
R₅ is hydrogen, alkyl of 1 to 8 carbon atoms or 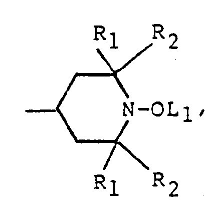
where L₁ is a monovalent radical of the definition of L,
p is O to 1O, q is 2 to 4,
R₆ is hydrogen or alkyl of 1 to 18 carbon atoms,
R₇ is hydrogen or -CH₂CH₂COOC₁₂H₂₅,
D is a diradical of an alkane or alkene of 1 to 18 carbon atoms, diradical of a cycloalkane or cycloalkene of 5 to 12 carbon atoms, or a diradical of a saturated or unsaturated bicyclic or tricyclic hydrocarbon of 7 to 12 carbon atoms, with the proviso that the N-O groups are not necessarily attached to the same carbon atom in D,
E is a monovalent radical of an alkane or alkene of 1 to 18 carbon atoms, a monovalent radical of a cycloalkane or cycloalkene of 5 to 12 carbon atoms or a monovalent radical of a saturated or unsaturated bicyclic or tricyclic hydrocarbon of 7 to 12 carbon atoms,
G is an organic linking moiety selected from the group consisting of -OCO-R₈-COO-, -COO-R₉-OCO-, -NR₅-CO-R₈-CONR₅-, 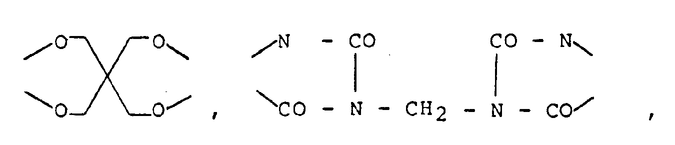 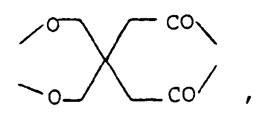 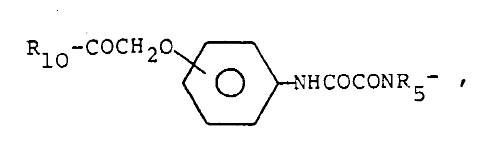 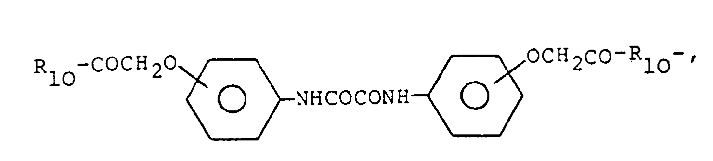
and 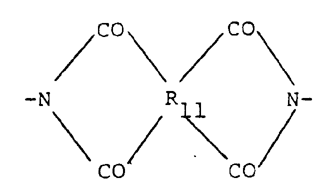
where
R₈ is a direct bond or a divalent radical of an aliphatic, cycloaliphatic, unsaturated aliphatic or aromatic dicarboxylic acid without the two carboxy groups,
R₉ is a divalent radical of an aliphatic, cycloaliphatic or unsaturated aliphatic diol without the two hydroxy groups,
R_{1O} is -O- or -NR₅-, and
R₁₁ is an aliphatic or aromatic tetravalent radical.
Preferably in formula I m is 2 to 4, especially 2.
Preferably in formula II n is 1 or 2.
Preferably R₁ and R₂ are each methyl.
Preferably in formula I L is an m-valent radical of n-octane, n-heptane or cyclohexane.
Preferably in formula I T is R₃-COO- where R₃ is phenyl, vinyl or alkyl of 1 to 17 carbon atoms, most preferably heptadecyl, or T in formula I preferably is R₃-CONR₅- where R₅ is hydrogen and R₃ is vinyl, or T is 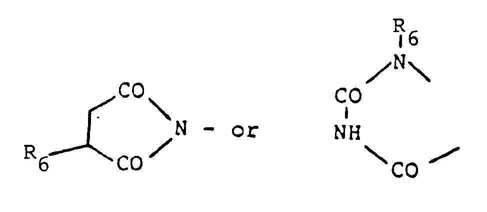
where R₆ is dodecyl.

Preferably in formula II D is a diradical of n-octane, n-heptane or cyclohexane.

Preferably in formula II E is octyl, heptyl or cyclohexyl.

Preferably in formula II G is -OCO-R₈-COO- where R₈ is alkylene of 2 to 8 carbon atoms, most preferably 2 or 8 carbon atoms, or phenylene, or
G in formula II is preferably -NR₅-CO-R₈-CONR₅- where R₅ is hydrogen or butyl and R₈ is a direct bond or alkylene of 2 to 8 carbon atoms, most preferably a direct bond, ethylene or octamethylene, or
G is

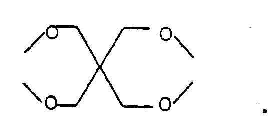

Monomeric N-hydrocarbyloxy derivatives of hindered amines can be made by a variety of synthetic routes. These include:
a. reaction of an N-oxyl compound with an alkyl halide in the presence of tri-n-butyltin hydride (R. L. Kinney et al, J. Am. Chem. Soc. 100, 7902 (1978));
b. reaction of an N-hydroxy compound with an alkyl halide and n-butyllithium or sodium hydride (T. Kurumada et al, J. Poly Sci, Poly Chem. Ed, 22, 277 (1984) and 23, 1477 (1985));
c. The photolysis of a solution of an N-oxyl compound, a hydrocarbon and di-tert-butyl peroxide (D. W. Grattan et al, Polym. Degrad & Stability, 1979, 69);
d. The thermolysis of a solution of an N-oxyl compound, a hydrocarbon and a tert-butyl perester (A. J. Beckwith, J. Org. Chem. 53, 1632 (1988));
e. The photolysis of a solution of an N-oxyl compound, a hydrocarbon and tert-butyl hydroperoxide in an oxygen atmosphere (T. Kurumada et al, J. Polym. Sci, Polym Chem. Ed, 23, 1477 (1985)); and
f. The thermolysis of a solution of a hindered amine or an N-oxyl hindered amine, a hydrocarbon, tert-butyl hydroperoxide and a metal oxide catalyst (EP-A-3O9 4O2).

The instant oligomeric compounds can be prepared by modifications of the methods set forth supra for preparing monomeric N-hydrocarbyloxy compounds.

For example the compounds of formula I can be prepared by reaction of a N-oxyl or N-hydroxy hindered amine with a polyhaloalkane.

Most conveniently the compounds of formula I are prepared by coupling a N-oxyl hindered amine with a hydrocarbon n-valent radical generated from the decomposition of a peroxide or hydroperoxide in the presence of a hydrocarbon having abstractable hydrogen atoms.

The ratio of N-oxyl hindered amine to hydrocarbon can be adjusted to favor the formation of monomeric N-hydrocarbyloxy compounds or the formation of the instant polysubstituted N-hydrocarbyloxy compounds, but mixtures of monomeric and polysubstituted N-hydrocarbyloxy compounds are nearly always obtained. These mixtures can be easily separated into monomeric and polysubstituted N-hydrocarbyloxy compounds by column chromatography.

Thus, the group L can be assigned a specific structure when the instant compounds are made using the latter method, but the group L represents an isomeric mixture of structures when the instant compounds are made using an N-oxyl compound and a hydrocarbon with hydroperoxide or peroxide.

The compounds of formula II can be synthesized by reaction of bis(N-oxyl) compounds with hydrocarbon radicals generated from the decomposition of a peroxide or hydroperoxide in the presence of a hydrocarbon solvent with abstractable hydrogen atoms. At sufficiently low concentrations of solvent, two N-oxyl molecules can react with the same solvent molecule, but mixtures of monomeric and oligomeric N-hydrocarbyloxy compounds are nearly always obtained. These mixtures can be easily separated into monomeric and oligomeric N-hydrocarbyloxy compounds by column chromatography.

Bis(N-oxyl) hindered amines can further be reacted with tributyltin hydride and aliphatic dihalides, preferably diiodides, to give oligomeric products of formula II. Bis(N-hydroxy) hindered amines can be alkylated using a suitable base and aliphatic dihalides. These methods are expected to give mixtures of oligomers of different molecular weights.

Any terminal halogenated N-hydrocarbyloxy groups can be reduced with tributyltin hydride using standard methodology.

Oligomers of formula II having specific molecular weights can be synthesized by preparing difunctional hindered amine intermediates which have one N-hydrocarbyloxy group or bridge and one N-oxyl or N-hydroxy functionality. These intermediates can be coupled using the aliphatic dihalide chemistry discussed in the preceding paragraph.

Although the instant application emphasizes the 2,2,6,6-tetralkylpiperidine structure, it is to be noted that the invention also relates to compounds wherein the following tetraalkyl substituted piperazine or piperazinone moieties are substituted for the above-noted tetraalkylpiperidine moiety: 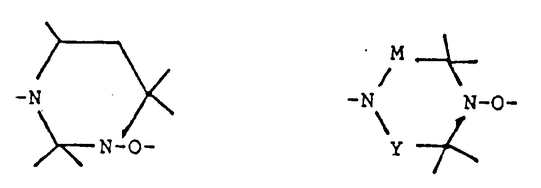
wherein M and Y are independently methylene or carbonyl, preferably M being methylene and Y being carbonyl. It is understood that the identified substituents applicable to such compounds are those which are appropriate for substitution on the ring nitrogen atoms.

Substrates in which the compounds of this invention are particularly useful are polyolefins such as polyethylene and polypropylene; polystyrene, including especially impact polystyrene; ABS resin; elastomers such as e.g. butadiene rubber, EPM, EPDM, SBR and nitrile rubber.

In general polymers which can be stabilized include
1. Polymers of monoolefins and diolefins, for example polyethylene (which optionally can be crosslinked), polypropylene, polyisobutylene, polybutene-1, polymethylpentene-1, polyisoprene or polybutadiene, as well as polymers of cycloolefins, for instance of cyclopentene or norbornene.
2. Mixtures of the polymers mentioned under 1), for example mixtures of polypropylene with polyisobutylene.
3. Copolymers of monoolefins and diolefins with each other or with other vinyl monomers, such as, for example, ethylene/propylene, propylene/butene-1, propylene/isobutylene, ethylene/butene-1, propylene/butadiene, isobutylene/isoprene, ethylene/alkyl acrylates, ethylene/alkyl methacrylates, ethylene/vinyl acetate or ethylene/acrylic acid copolymers and their salts (ionomers) and terpolymers of ethylene with propylene and a diene, such as hexadiene, dicyclopentadiene or ethylidene-norbornene.
4. Polystyrene, poly-(p-methylstyrene).
5. Copolymers of styrene or methylstyrene with dienes or acrylic derivatives, such as, for example, styrene/butadiene, styrene/acrylonitrile, styrene/ethyl methacrylate, styrene/butadiene/ethyl acrylate, styrene/acrylonitrile/methyl acrylate; mixtures of high impact strength from styrene copolymers and another polymer, such as, for example, from a polyacrylate, a diene polymer or an ethylene/propylene/diene terpolymer; and block polymers of styrene, such as, for example, styrene/butadiene/styrene, styrene/isoprene/styrene, styrene/ethylene/butylene/styrene or styrene/ethylene/propylene/styrene.
6. Graft copolymers of styrene, such as, for example, styrene on polybutadiene, styrene and acrylonitrile on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS-, MBS-, ASA- or AES-polymers.
7. Halogen-containing polymers, such as polychloroprene, chlorinated rubbers, chlorinated or sulfochlorinated polyethylene, epichlorohydrin homo- and copolymers, polymers from halogen-containing vinyl compounds, as for example, polyvinylchloride, polyvinylidene chloride, polyvinyl fluoride, polyvinylidene fluoride, as well as copolymers thereof, as for example, vinyl chloride/vinylidene chloride, vinyl chloride/vinyl acetate, vinylidene chloride/vinyl acetate copolymers, or vinyl fluoride/vinyl ether copolymers.
8. Polymers which are derived from α,β-unsaturated acids and derivatives thereof, such as polyacrylates and polymethacrylates, polyacrylamide and polyacrylonitrile.
9. Copolymers from the monomers mentioned under 8) with each other or with other unsaturated monomers, such as, for instance, acrylonitrile/butadiene, acrylonitrile/alkyl acrylate, acrylonitrile/alkoxyalkyl acrylate or acrylonitrile/vinyl halogenide copolymers or acrylonitrile/alkyl methacrylate/butadiene terpolymers.
10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinylbutyral, polyallyl phthalate or polyallyl-melamine.
11. Homopolymers and copolymers of cyclic ethers, such as polyalkylene glycols, polyethylene oxide, polypropylene oxide or copolymers thereof with bis-glycidyl ethers.
12. Polyacetals, such as polyoxymethylene and those polyoxymethylenes which contain ethylene oxide as comonomer.
13. Polyphenylene oxides and sulfides, and mixtures of polyphenylene oxides with polystyrene.
14. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).
15. Polyamides and copolyamides which are derived from diamines and dicarboxylic acids and/or from aminocarboxylic acids or the corresponding lactams, such as polyamide 4, polyamide 6, polyamide 6/6, polyamide 6/10, polyamide 11, polyamide 12, poly-2,4,4-trimethylhexamethylene terephthalamide, poly-p-phenylene terephthalamide or poly-m-phenylene isophthalamide, as well as copolymers thereof with polyethers, such as for instance with polyethylene glycol, polypropylene glycol or polytetramethylene glycols.
16. Polyureas, polyimides and polyamide-imides.
17. Polyesters which are derived from dicarboxylic acids and diols and/or from hydroxycarboxylic acids or the corresponding lactones, such as polyethylene terephthalate, polybutylene terephthalate, poly-1,4-dimethylol-cyclohexane terephthalate, poly-[2,2-(4-hydroxyphenyl)-propane] terephthalate and polyhydroxybenzoates as well as block-copolyether-esters derived from polyethers having hydroxyl end groups.
18. Polycarbonates.
19. Polysulfones, polyethersulfones and polyetherketones.
20. Crosslinked polymers which are derived from aldehydes on the one hand and phenols, ureas and melamines on the other hand, such as phenol/formaldehyde resins, urea/formaldehyde resins and melamine/formaldehyde resins.
21. Drying and non-drying alkyd resins.
22. Unsaturated polyester resins which are derived from copolyesters of saturated and unsaturated dicarboxylic acids with polyhydric alcohols and vinyl compounds as crosslinking agents, and also halogen-containing modifications thereof of low flammability.
23. Thermosetting acrylic resins, derived from substituted acrylic esters, such as epoxy-acrylates, urethane-acrylates or silicone -acrylates.
24. Alkyd resins, polyester resins or acrylate resins in admixture with melamine resins, urea resins, polyisocyanates or epoxide resins as crosslinking agents.
25. Crosslinked epoxide resins which are derived from polyepoxides, for example from bis-glycidyl ethers or from cycloaliphatic diepoxides.
26. Natural polymers, such as cellulose, rubber, gelatin and derivatives thereof which are chemically modified in a polymer homologous manner, such as cellulose acetates, cellulose propionates and cellulose butyrates, or the cellulose ethers, such as methyl cellulose.
27. Mixtures of polymers as mentioned above, for example PP/EPDM, Polyamide 6/EPDM or ABS, PVC/EVA, PVC/ABS, PVC/MBS, PC/ABS, PBTP/ABS.
28. Naturally occuring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellitates) and also mixtures of synthetic esters with mineral oils in any weight ratios, which materials may be used as plasticizers for polymers or as textile spinning oils, as well as aqueous emulsions of such materials.
29. Aqueous emulsions of natural or synthetic rubber, e.g. natural latex or latices of carboxylated styrene/butadiene copolymers.
30. Polysiloxanes such as the soft, hydrophilic polysiloxanes described, for example, in U.S. Patent No. 4,259,467; and the hard polyorganosiloxanes described, for example, in U.S. Patent No. 4,355,147.
31. Polyketimines in combination with unsaturated acrylic polyacetoacetate resins or with unsaturated acrylic resins. The unsaturated acrylic resins include the urethane acrylates, polyether acrylates, vinyl or acryl copolymers with pendant unsaturated groups and the acrylated melamines. The polyketimines are prepared from polyamines and ketones in the presence of an acid catalyst.
32. Radiation curable compositions containing ethylenically unsaturated monomers or oligomers and a polyunsaturated aliphatic oligomer.
33. Epoxymelamine resins such as light-stable epoxy resins crosslinked by an epoxy functional coetherified high solids melamine resin such as LSE 4103 (Monsanto).

In general, the compounds of the present invention are employed in from about 0.01 to about 5% by weight of the stabilized composition, although this will vary with the particular substrate and application. An advantageous range is from about 0.5 to about 2%, and especially 0.1 to about 1%.

The stabilizers of the instant invention may readily be incorporated into the organic polymers by conventional techniques, at any convenient stage prior to the manufacture of shaped articles therefrom. For example, the stabilizer may be mixed with the polymer in dry powder form, or a suspension or emulsion of the stabilizer may be mixed with a solution, suspension, or emulsion of the polymer. The resulting stabilized polymer compositions of the invention may optionally also contain various conventional additives, such as the following.

### 1. Antioxidants

### 1.1. Alkylated monophenols, for example,

2,6-di-tert-butyl-4-methylphenol
2-tert-butyl-4,6-dimethylphenol
2,6-di-tert-butyl-4-ethylphenol
2,6-di-tert-butyl-4-n-butylphenol
2,6-di-tert-butyl-4-i-butylphenol
2,6-di-cyclopentyl-4-methylphenol
2-(α-methylcyclohexyl)-4,6-dimethylphenol
2,6-di-octadecyl-4-methylphenol
2,4,6-tri-cyclohexylphenol
2,6-di-tert-butyl-4-methoxymethylphenol

### 1.2. Alkylated hydroquinones, for example,

2,6-di-tert-butyl-4-methoxyphenol
2,5-di-tert-butyl-hydroquinone
2,5-di-tert-amyl-hydroquinone
2,6-diphenyl-4-octadecyloxyphenol

### 1.3. Hydroxylated thiodiphenyl ethers, for example

2,2'-thio-bis-(6-tert-butyl-4-methylphenol)
2,2'-thio-bis-(4-octylphenol)
4,4'-thio-bis-(6-tert-butyl-3-methylphenol)
4,4'-thio-bis-(6-tert-butyl-2-methylphenol)

### 1.4. Alkylidene-bisphenols, for example,

2,2'-methylene-bis-(6-tert-butyl-4-methylphenol)
2,2'-methylene-bis-(6-tert-butyl-4-ethylphenol)
2,2'-methylene-bis-[4-methyl-6-(α-methylcyclohexyl)-phenol]
2,2'-methylene-bis-(4-methyl-6-cyclohexylphenol)
2,2'-methylene-bis-(6-nonyl-4-methylphenol)
2,2'-methylene-bis-[6-(α-methylbenzyl)-4-nonylphenol]
2,2'-methylene-bis-[6-(α,α-dimethylbenzyl)-4-nonylphenol]
2,2'-methylene-bis-(4,6-di-tert-butylphenol)
2,2'-ethylidene-bis-(4,6-di-tert-butylphenol)
2,2'-ethylidene-bis-(6-tert-butyl-4-isobutylphenol)
4,4'-methylene-bis-(2,6-di-tert-butylphenol)
4,4'-methylene-bis-(6-tert-butyl-2-methylphenol)
1,1-bis-(5-tert-butyl-4-hydroxy-2-methylphenyl-butane
2,6-di-(3-tert-butyl-5-methyl-2-hydroxybenzyl)-4-methylphenol
1,1,3-tris-(5-tert-butyl-4-hydroxy-2-methylphenyl)-butane
1,1-bis-(5-tert-butyl-4-hydroxy-2-methylphenyl)-3-n-dodecylmercaptobutane
ethyleneglycol bis-[3,3-bis-(3'-tert-butyl-4'-hydroxyphenyl)-butyrate]
di-(3-tert-butyl-4-hydroxy-5-methylphenyl)-dicyclopentadiene
di-[2-(3'-tert-butyl-2'-hydroxy-5'-methyl-benzyl)-6-tert-butyl-4-methylphenyl] terephthalate.

### 1.5. Benzyl compounds, for example,

1,3,5-tri-(3,5-di-tert-butyl-4-hydroxybenzyl)-2,4,6-trimethylbenzene
di-(3,5-di-tert-butyl-4-hydroxybenzyl) sulfide
3,5-di-tert-butyl-4-hydroxybenzyl-mercapto-acetic acid isooctyl ester
bis-(4-tert-butyl-3-hydroxy-2,6-dimethylbenzyl)dithiol terephthalate
1,3,5-tris-(3,5-di-tert-butyl-4-hydroxybenzyl) isocyanurate
1,3,5-tris-(4-tert-butyl-3-hydroxy-2,6-dimethylbenzyl) isocyanurate
3,5-di-tert-butyl-4-hydroxybenzyl-phosphoric acid dioctadecyl ester
3,5-di-tert-butyl-4-hydroxybenzyl-phosphoric acid monoethyl ester, calcium-salt

### 1.6. Acylaminophenols, for example,

4-hydroxy-lauric acid anilide
4-hydroxy-stearic acid anilide
2,4-bis-octylmercapto-6-(3,5-tert-butyl-4-hydroxyanilino)-s-triazine
octyl-N-(3,5-di-tert-butyl-4-hydroxyphenyl)-carbamate

### 1.7. Esters of β-(3,5-di-tert-butyl-4-hydroxyphenyl)-propionic acid with monohydric or polyhydric alcohols, for example,

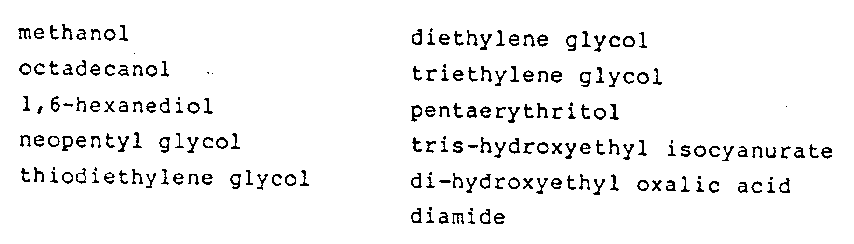

### 1.8. Esters of β-(5-tert-butyl-4-hydroxy-3-methylphenyl)-propionic acid with monohydric or polyhydric alcohols, for example,

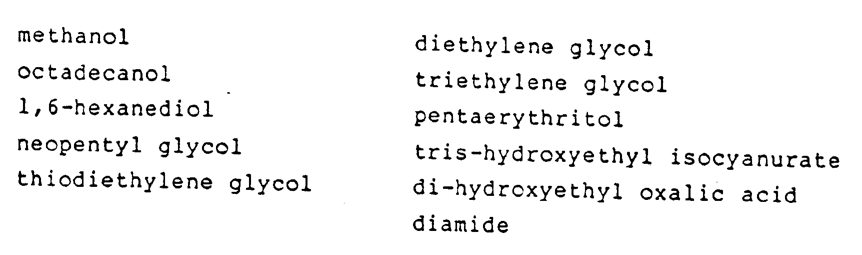

### 1.9. Amides of β-(3,5-di-tert-butyl-4-hydroxyphenyl)-propionic acid for example,

N,N'-di-(3,5-di-tert-butyl-4-hydroxyphenylpropionyl)-hexamethylenediamine
N,N'-di-(3,5-di-tert-butyl-4-hydroxyphenylpropionyl)-trimethylenediamine
N,N'-di-(3,5-di-tert-butyl-4-hydroxyphenylpropionyl)-hydrazine

### 2. UV absorbers and light stabilizers

2.1. 2-(2'-Hydroxyphenyl)-benzotriazoles, for example, the 5'-methyl-, 3',5'-di-tert-butyl-, 5'-tert-butyl-, 5'-(1,1,3,3-tetramethylbutyl)-, 5-chloro-3',5'-di-tert-butyl-, 5-chloro-3'-tert-butyl-5'-methyl-, 3'-sec-butyl-5'-tert-butyl-, 4'-octoxy, 3',5'-di-tert-amyl-, 3',5'-bis-(α,α-dimethylbenzyl), 3'-tert-butyl-5'-(2-(omega-hydroxy-octa-(ethyleneoxy)carbonyl-ethyl)-, 3'-dodecyl-5'-methyl-, and 3'-tert-butyl-5'-(2-octyloxycarbonyl)ethyl-, and dodecylated-5'-methyl derivatives.
2.2. 2-Hydroxy-benzophenones, for example, the 4-hydroxy-, 4-methoxy-, 4-octoxy, 4-decyloxy-, 4-dodecyloxy-, 4-benzyloxy, 4,2',4'-trihydroxy- and 2'-hydroxy-4,4'-dimethoxy derivatives.
2.3. Esters of optionally substituted benzoic acids for example, phenyl salicylate, 4-tert-butylphenyl salicylate, octylphenyl salicylate, dibenzoylresorcinol, bis-(4-tert-butylbenzoyl)-resorcinol, benzoylresorcinol, 3,5-di-tert-butyl-4-hydroxybenzoic acid 2,4-di-tert-butylphenyl ester and 3,5-di-tert-butyl-4-hydroxybenzoic acid hexadecyl ester.
2.4. Acrylates, for example, α-cyano-β,β-diphenylacrylic acid ethyl ester or isooctyl ester, α-carbomethoxy-cinnamic acid methyl ester, α-cyano-β-methyl-p-methoxy-cinnamic acid methyl ester or butyl ester, α-carbomethoxy-p-methoxy-cinnamic acid methyl ester, N-(β-carbomethoxy-β-cyanovinyl)-2-methyl-indoline.
2.5 Nickel compounds, for example, nickel complexes of 2,2'-thio-bis-[4-(1,1,3,3-tetramethylbutyl)-phenol], such as the 1:1 or 1:2 complex, optionally with additional ligands such as n-butylamine, triethanolamine or N-cyclohexyl-diethanolamine, nickel dibutyldithiocarbamate, nickel salts of 4-hydroxy-3,5-di-tert-butylbenzylphosphonic acid monoalkyl esters, such as of the methyl, ethyl or butyl ester, nickel complexes of ketoximes such as of 2-hydroxy-4-methyl-phenyl undecyl ketoxime, nickel complexes of 1-phenyl-4-lauroyl-5-hydroxy-pyrazole, optionally with additional ligands.
2.6. Sterically hindered amines, for example bis-(2,2,6,6-tetramethylpiperidyl) sebacate, bis-(1,2,2,6,6-pentamethylpiperidyl) sebacate, n-butyl-3,5-di-tert.butyl-4-hydroxybenzyl malonic acid bis-(1,2,2,6,6-pentamethylpiperidyl)ester, condensation product of 1-hydroxyethyl-2,2,6,6-tetramethyl-4-hydroxypiperidine and succinic acid, condensation product of N,N'-(2,2,6,6-tetramethylpiperidyl)-hexamethylenediamine and 4-tert-octylamino-2,6-dichloro-s-triazine, tris-(2,2,6,6-tetramethylpiperidyl)-nitrilotriacetate, tetrakis-(2,2,6,6-tetramethyl-4-piperidyl)-1,2,3,4-butane-tetracarbonic acid, 1,1'(1,2-ethanediyl)-bis-(3,3,5,5-tetramethylpiperazinone).
2.7. Oxalic acid diamides, for example, 4,4'-di-octyloxyoxanilide, 2,2'-di-octyloxy-5,5'-di-tert-butyl-oxanilide, 2,2'-di-dodecyloxy-5,5'-di-tert-butyl-oxanilide, 2-ethoxy-2'-ethyl-oxanilide, N,N'-bis (3-dimethylaminopropyl)-oxalamide, 2-ethoxy-5-tert-butyl-2'-ethyloxanilide and its mixture with 2-ethoxy-2'-ethyl-5,4'-di-tert-butyloxanilide and mixtures of ortho- and para-methoxy-as well as of o-and p-ethoxy-disubstituted oxanilides.
2.8. Hydroxyphenyl-s-triazines, for example 2,6-bis-(2,4-dimethylphenyl)-4-(2-hydroxy-4-octyloxyphenyl)-s-triazine; 2,6-bis-(2,4-dimethylphenyl)-4-(2,4-dihydroxyphenyl)-s-triazine; 2,4-bis(2,4-dihydroxyphenyl)-6-(4-chlorophenyl)-s-triazine; 2,4-bis[2-hydroxy-4-(2-hydroxyethoxy)phenyl]-6-(4-chlorophenyl)-s-triazine; 2,4-bis[2-hydroxy-4-(2-hydroxyethoxy)phenyl]-6-phenyl-s-triazine; 2,4-bis[2-hydroxy-4-(2-hydroxyethoxy)phenyl]-6-(2,4-dimethylphenyl)-s-triazine; 2,4-bis[2-hydroxy-4-(2-hydroxyethoxy)phenyl]-6-(4-bromophenyl)-s-triazine; 2,4-bis[2-hydroxy-4-(2-acetoxyethoxy)phenyl]-6-(4-chlorophenyl)-s-triazine, 2,4-bis(2,4-dihydroxyphenyl)-6-(2,4-dimethylphenyl)-s-triazine.
3. Metal deactivators, for example, N,N'-diphenyloxalic acid diamide, N-salicylal-N'-salicyloylhyrazine, N,N'-bis-salicyloylhydrazine, N,N'-bis-(3,5-di-tert-butyl-4-hydroxyphenylpropionyl)-hydrazine, 3-salicyloylamino-1,2,4-triazole, bis-benzylidene-oxalic acid dihydrazide.
4. Phosphites and phosphonites, for example, triphenyl phosphite, diphenylalkyl phosphites, phenyldialkyl phosphites, tri-(nonylphenyl) phosphite, trilauryl phosphite, trioctadecyl phosphite, di-stearyl-pentaerythritol diphosphite, tris-(2,4-di-tert-butylphenyl) phosphite, di-isodecylpentaerythritol diphosphite, di-(2,4-di-tert-butylphenyl)pentaerythritol diphosphite, tristearylsorbitol triphosphite, tetrakis-(2,4-di-tert-butylphenyl)-4,4'-diphenylylenediphosphonite.
5. Compounds which destroy peroxide, for example, esters of β-thiodipropionic acid, for example the lauryl, stearyl, myristyl or tridecyl esters, mercapto-benzimidazole or the zinc salt of 2-mercaptobenzimidazole, zinc dibutyl-dithiocarbamate, dioctadecyl disulfide, pentaerythritol tetrakis-(β-dodecylmercapto)-propionate.
6. Hydroxylamines, for example, N,N-dibenzylhydroxylamine, N,N-diethylhydroxylamine, N,N-dioctylhydroxylamine, N,N-diluarylhydroxylamine, N,N-ditetradecylhydroxylamine, N,N-dihexadecylhydroxylamine, N,N-dioctadecylhydroxylamine, N-hexadecyl-N-octadecylhydroxylamine, N-heptadecyl-N-octadecylhydroxylamine, N,N-dialkylhydroxylamine derived from hydrogenated tallow amine.
7. Polyamide stabilizers, for example copper salts in combination with iodides and/or phosphorus compounds and salts of divalent manganese.
8. Basic co-stabilizers, for example, melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example Ca stearate, Zn stearate, Mg stearate, Na ricinoleate and K palmitate, antimony pyrocatecholate or zinc pyrocatecholate.
9. Nucleating agents, for example, 4-tert-butyl-benzoic acid, adipic acid, diphenylacetic acid.
10. Fillers and reinforcing agents, for example, calcium carbonate, silicates, glass fibers, asbestos, talc, kaolin, mica, barium sulfate, metal oxides and hydroxides, carbon black, graphite.
11. Other additives, for example, plasticizers, lubricants, emulsifiers, pigments, optical brighteners, flameproofing agents, anti-static agents, blowing agents and thiosynergists such as dilauryl thiodipropionate or distearyl thiodipropionate.

Of particular interest is the utilization of the instant derivatives in a variety of coating systems including ambient cured and acid catalyzed coating systems. In particular, the physical integrity of the coatings is maintained to a higher degree with significant reduction in loss of gloss and yellowing. Key improvements include the substantial absence of the cure retardation encountered with N-alkyl hindered amine light stabilizers; the substantial absence of flocculation and dispersion destabilization seen when N-alkyl hindered amines are utilized in certain pigmented coating systems and the absence of adhesion loss between the coating and polycarbonate substrate. Accordingly, the present invention also relates to the use of the instant compounds, optionally together with further stabilizers, for stabilizing ambient cured coatings based on alkyd resins; thermoplastic acrylic resins; acrylic alkyds; acrylic alkyd or polyester resins optionally modified with silicon, isocyanates, isocyanurates, ketimines or oxazolidines; and epoxide resins crosslinked with carboxylic acids, anhydrides, polyamines or mercaptans; and acrylic and polyester resin systems modified with reactive groups in the backbone thereof and crosslinked with epoxides; against the degradative effects of light, moisture and oxygen.

Furthermore, in their industrial uses, enamels with high solids content based on crosslinkable acrylic, polyester, urethane or alkyd resins are cured with an additional acid catalyst. Light stabilizers containing a basic nitrogen group are generally less than satisfactory in this application. Formation of a salt between the acid catalyst and the light stabilizer leads to incompatibility or insolubility and precipitation of the salt and to a reduced level of cure and to reduced light protective action and poor resistance to moisture.

These acid catalyzed stoving lacquers are based on hot crosslinkable acrylic, polyester, polyurethane, polyamide or alkyd resins. The acrylic resin lacquers, which can be stabilized against light, moisture and oxygen in accordance with the invention, are the conventional acrylic resin stoving lacquers or thermosetting resins including acrylic/melamine systems which are described, for example, in H. Kittel's "Lehrbuch der Lacke und Beschichtungen", Vol. 1 Par 2, on pages 735 and 742 (Berlin 1972), "Lackkunstharze" (1977), by H. Wagner and H.F. Sarx, on pages 229-238, and in S. Paul's "Surface Coatings: Science and Technology" (1985).

The polyester lacquers, which can be stabilized against the action of light and moisture, are the conventional stoving lacquers described e.g. in H. Wagner and H.F. Sarx, op. cit., on pages 86-99.

The alkyd resin lacquers which can be stabilized against the action of light and moisture in accordance with the invention, are the conventional stoving lacquers which are used in particular for coating automobiles (automobile finishing lacquers), for example lacquers based on alkyd/melamine resins and alkyd/acrylic/melamine resins (see H. Wagner and H. F. Sarx, op. cit., pages 99-123). Other crosslinking agents include glycoluril resins, blocked isocyanates or epoxy resins.

The acid catalyzed stoving lacquers stabilized in accordance with the invention are suitable both for metal finish coatings and solid shade finishes, especially in the case of retouching finishes, as well as various coil coating applications. The lacquers stabilized in accordance with the invention are preferably applied in the conventional manner by two methods, either by the single-coat method or by the two-coat method. In the latter method, the pigment-containing base coat is applied first and then a covering coat of clear lacquer over it.

It is also to be noted that the instant substituted hindered amines are applicable for use in non-acid catalyzed thermoset resins such as epoxy, epoxy-polyester, vinyl, alkyd, acrylic and polyester resins, optionally modified with silicon, isocyanates or isocyanurates. The epoxy and epoxy-polyester resins are crosslinked with conventional crosslinkers such as acids, acid anhydrides, amines, and the like.

Correspondingly, the epoxide may be utilized as the crosslinking agent for various acrylic or polyester resin systems that have been modified by the presence of reactive groups on the backbone structure.

To attain maximum light stability in such coatings, the concurrent use of other conventional light stabilizers can be advantageous. Examples are the aforementioned UV absorbers of the benzophenone, benzotriazole, acrylic acid derivative, or oxanilide type, or aryl-s-triazines or metal-containing light stabilizers, for example organic nickel compounds. In two-coat systems, these additional light stabilizers can be added to the clear coat and/or the pigmented base coat.

If such combinations are employed, the sum of all light stabilizers is 0.2 to 20% by weight, preferably 0.5 to 5% by weight, based on the film-forming resin.

Examples of different classes of UV absorbers which may be used in the instant compositions in conjunction with the aforementioned piperidine compounds are reference in a paper by H. J. Heller in European Polymer Journal Supplement, 1969, pp 105-132. These classes include the phenyl salicylates, the o-hydroxybenzopheones, the hydroxyxanthones, the benzoxazoles, the benzimidazoles, the oxadizaoles, the triazoles, the pyrimidines, the chinazolines, the s-triazines, the hydroxyphenyl-benzotriazoles, the alpha-cyanoacrylates and the benzoates.

Types of UV absorbers of especial importance are:
(a) 2-(2'-Hydroxyphenyl)-benzotriazoles, for example, the 5'-methyl-, 3',5'-di-tert-butyl-, 5'-tert-butyl-, 5'-(1,1,3,3-tetramethylbutyl)-, 5-chloro-3'-5'-di-tert-butyl-, 5-chloro-3'-tert-butyl-5'-methyl-, 3'-sec-butyl-5'-tert-butyl-, 4'-octoxy-, and 3',5'-di-tert-amyl derivatives.
(b) 2-Hydroxy-benzophenones, for example, the 4-hydroxy-, 4-methoxy-, 4-octoxy-, 4-decyloxy-, 4-dodecyloxy-, 4-benzyloxy, 4,2',4'-trihydroxy- and 2'-hydroxy-4,4'-dimethoxy derivatives.
(c) Acrylates, for example, alpha-cyano-β,β-diphenylacrylic acid ethyl ester or isoctyl ester, alpha-carbomethoxy-cinnamic acid methyl ester, alpha-cyano-β-methyl-p-methoxy-cinnamic acid methyl ester or butyl ester, alpha-carbomethoxy-p-methoxy-cinnamic acid methyl ester, N-(β-carbomethoxy-β-cyanovinyl)-2-methyl-indoline.
(d) Nickel compounds, for example, nickel complexes of 2,2'-thiobis-[4-(1,1,3,3-tetramethylbutyl)-phenol], such as the 1:1 or 1:2 complex, optionally with additional ligands such as n-butylamine, triethanolamine or N-cyclohexyl-diethanolamine, nickel dibutyldithiocarbamate, nickel salts of 4-hydroxy-3,5-di-tert-butylbenzylphosphonic acid monoalkyl esters, such as of the methyl, ethyl or butyl ester, nickel complexes of ketoximes such as of 2-hydroxy-4-methyl-phenyl undecyl ketonoxime, nickel complexes of 1-phenyl-4-lauroyl-5-hydroxy-pyrazol, optionally with additional ligands.
(e) Oxalic acid diamides, for example, 4,4'-di-octyl-oxyoxanilide, 2,2'-di-octyloxy-5,5'-di-tert-butyl-oxanilide, 2,2'-di-dodecyloxy-5,5'-di-tert-butyl-oxanilide, 2-ethoxy-2'-ethyl-oxanilide, N,N'-bis-(3-dimethylaminopropyl)-oxalamide, 2-ethoxy-5-tert-butyl-2'-ethyl-oxanilide and its mixture with 2-ethoxy-2'-ethyl-5,4'-di-tert-butyl-oxanilide and its mixtures of ortho- and para-methoxy- as well as of o- and p-ethoxy-disubstituted oxanilides.
(f) Hydroxyphenyl-s-triazines such as 2,6-bis(2,4-dimethylphenyl)-4-(2-hydroxy-4-octyloxyphenyl)-s-triazine or the correspsonding 4-(2,4-dihydroxyphenyl) derivative.

Of particular value in the instant compositions are the benzotriazoles of high molecular weight and low volatility such as 2-[2-hydroxy-3,5-di(alpha,alpha-dimethylbenzyl)-phenyl]-2H-benzotriazole, 2-(2-hydroxy-3,5-di-tert-octylphenyl)-2H-benzotriazole, 2-(2-hydroxy-3-alpha,alpha-dimethylbenzyl-5-tert-octyl-phenyl)-2H-benzotriazole, 2-(2-hydroxy-3-tert-octyl-5-alpha,alpha-dimethylbenzylphenyl)-2H-benzotriazole, 2-(2-hydroxy-3,5-di-tert-amylphenyl)-2H-benzotriazole, 2-[2-hydroxy-2-tert-butyl-5-(2-(omega-hydroxy-octa-(ethyleneoxy)carbonyl)-ethylphenyl]-2H-benzotriazole, dodecylated 2-(2-hydroxy-5-methylphenyl)-2H-benzotriazole, 2-[2-hydroxy-3-tert.butyl-5-(2-octyloxycarbonyl)ethylphenyl]-2H-benzotriazole and the 5-chloro compounds corresponding to each of the above named benzotriazoles.

Most preferably the benzotriazoles useful in the instant compositions are 2-[2-hydroxy-3,5-di(alpha,alpha-dimethyl-benzyl)phenyl]-2H-benzotriazole, dodecylated 2-(2-hydroxy-5-methylphenyl)-2H-benzotriazole, 2-[2-hydroxy-3-tert-butyl-5-(2-omega-hydroxy-octa-(ethyleneoxy)carbonyl)-ethylphenyl]-2H-benzotriazole, 2-[2-hydroxy-3-tert-butyl-5-(2-octyloxycarbonyl)ethylphenyl]-2H-benzotriazole and 5-chloro-2-[2-hydroxy-3-tert.butyl-5-(2-octyloxycarbonyl)ethylphenyl]-2H-benzotriazole.

It is also contemplated that the instant compounds will be particularly effective as stabilizers for polyolefin fibers, especially polypropylene fibers.

A preferred embodiment of the instant invention pertains to stabilized compositions comprising
(a) an acid catalyzed thermoset coating or enamel based on hot crosslinkable acrylic, polyester or alkyd resins,
(b) a compound of formula I or II, and
(c) a UV absorber selected from the group consisting of the benzophenones, benzotriazoles, acrylic acid derivatives, organic nickel compounds, aryl-s-triazines and oxanilides.

Further ingredients which the enamels or coatings can contain are antioxidants, for example those of the sterically hindered phenol derivatives, phosphorus compounds, such as phosphites, phosphines or phosphonites, plasticizers, levelling assistants, hardening catalysts, thickeners, dispersants or adhesion promoters.

A further preferred embodiment of the instant invention is a stabilized composition containing components (a), (b) and (c) described above which additionally contains as component (d) a phosphite or phosphonite.

The amount of phosphite or phosphonite (d) which is used in the instant compositions is from 0.05 to 2% by weight, preferably from 0.1 to 1% by weight, based on the film forming resin. In two-coat systems, these stabilizers may be added to the clear coat and/or base coat.

Typical phosphite and phosphonites include triphenyl phosphite, diphenylalkyl phosphites, phenyldialkyl phosphites, tri-(nonylphenyl)phosphite, trilauryl phosphite trioctadecyl phosphite, di-stearyl-pentaerythritol diphosphite, tris-(2,4-di-tert.butylphenyl) phosphite, di-isodecylpentaerythritol diphosphite, di-(2,4-di-tert.butylphenyl)pentaerythritol diphosphite, tristearyl-sorbitol triphosphite, tetrakis-(2,4-di-tert.butylphenyl)-4,4'-di-phenylylenediphosphonite.

The stabilizers are needed to impart greater retention of durability to the cured enamels (as measured by 20° gloss, distinction of image, cracking or chalking); the stabilizers must not retard cure (normal bake for auto finishes at 121°C and low bake repair at 82°C (as measured by hardness, adhesion, solvent resistance and humidity resistance), the enamel should not yellow on curing and further color change on exposure to light should be minimized; the stabilizers should be soluble in the organic solvents normally used in coating applications such as methyl amyl ketone, xylene, n-hexyl acetate, alcohol and the like.

The instant hindered amine light stabilizers substituted on the N-atom by an O-substituted moiety fulfill each of these requirements and provide alone or in combination with a UV-absorber outstanding light stabilization protection to the cured acid catalyzed thermoset enamels.

Still another preferred combination of the instant stabilizers is with a hydroxylamine in order to protect polypropylene fibers from gas fading.

### Example 1A

### 4-Benzoyloxy-1-(2-cyclohexen-1-yloxy)-2,2,6,6-tetramethylpiperidine

A solution of 33.6 grams (122 mmol) of 4-benzoyloxy-1-oxyl-2,2,6,6-tetramethylpiperidine, 23.0 grams (157 mmol) of di-tert-butyl peroxide, and 70 ml of cyclohexene is heated in a Fischer-Porter pressure bottle at 138°C for 6.5 hours. The reaction mixture is purified by flash chromatography on silica gel (200:1 heptane:ethyl acetate) to afford 35.1 grams (81% yield) of the title compound as a colorless oil.

### Analysis:

- Calcd for C₂₂H₃₁No₃:: C, 73.9; H, 8.7; N, 3.9.
- Found:: C, 73.7; H, 8.8; N, 3.9.

### Example 1B

### 1,4-Bis(4-benzoyloxy-2,2,6,6-tetramethylpiperidin-1-yloxy)-2-cyclohexene

A mixture of 43.6 grams (122 mmol) of the compound prepared in Example 1A, 40.5 grams (147 mmol) of 4-benzoyloxy-1-oxyl-2,2,6,6-tetramethylpiperidine, 17.8 grams (122 mmol) of di-tert-butyl peroxide and 50 ml of 1,2-dichlorobenzene is heated at 135°C for 5.5 hours in a Fisher-Porter pressure bottle. Fresh di-tert-butyl peroxide (8.0 grams, 55 mmol) is added and the reaction mixture is heated at 135°C for an additional three hours. The crude reaction mixture is purified by flash chromatography on silica gel (hexane; then 100:3 heptane:ethyl acetate) to afford 4.0 grams of the title compound as a while solid melting at 140-142°C.

### Analysis:

- Calcd for C₃₈H₅₂N₂O₆:: C, 72.1; H, 8.3; N, 4.4.
- Found:: C, 72,0; H, 8.5; N, 4.3.

### Example 2

### 1,4-Bis(4-benzoyloxy-2,2,6,6-tetramethylpiperidin-1-yloxy)-cyclohexane

The title compound is prepared by the catalytic hydrogenation of 1,4-bis(4-benzoyloxy-2,2,6,6-tetramethylpiperidin-1-yloxy)-2-cyclohexane prepared in Example 1B.

### Example 3

### Bis(4-benzoyloxy-2,2,6,6-tetramethylpiperidin-1-yloxy)octane

A mixture of 55.3 grams (0.2 mol) of 4-benzoyloxy-2,2,6,6-tetramethylpiperidine, 2.8 grams of molybdenum trioxide and 250 ml of n-octane is heated to 120°C. A solution of 90.2 grams (0.7 mol) of 70% aqueous tert-butyl hydroperoxide is added dropwise to the hot reaction mixture. Water is removed by azeotropic distillation and collected in a Dean-Stark trap. The reaction mixture is heated at reflux till the red color of the intermediate N-oxyl compound disappears. Solids are moved by filtration, and the filtrate is concentrated under vacuum to give an oil. Purification of the oil by flash chromatography on silica gel (100:3 heptane: ethyl acetate) affords 4-benzoyloxy-1-octyloxy-2,2,6,6-tetramethylpiperidine as a mixture of octyloxy isomers. Further elution of the chromatographic column with 50:3 heptane:ethyl acetate affords the title compound as a mixture of octanediyl isomers.

### Example 4

### Bis(4-hydroxy-2,2,6,6-tetramethylpiperidin-1-yloxy)octane

The title compound is prepared by basic hydrolysis (potassium hydroxide in ethanol) of the compound prepared in Example 3.

### Example 5

### 1,8-Bis(4-benzoyloxy-2,2,6,6-tetramethylpiperidin-1-yloxy)octane

This reaction is carried out under a nitrogen atmosphere. Tributyltin hydride (32.1 grams, 110 mmol) is added dropwise over a three-hour interval to a solution of 66.5 grams (241 mmol) of 4-benzoyloxy-1-oxyl-2,2,6,6-tetramethylpiperidine, 20.1 grams (54.9 mmol) of 1,8-diiodooctane and 160 ml of chlorobenzene. The solution is precooled to 10°C before addition of the tributyltin hydride begins. The reaction mixture is kept below 20°C throughout the addition, and is then stirred at room temperature for 17 hours. The red reaction mixture is passed through a column of silica gel (heptane, then 100:3 heptane:ethyl acetate). Fractions containing the desired product are concentrated to give 26.5 grams of a crude solid. Tributyltin iodide is removed by washing a solution of the crude solid with aqueous ammonia. Final purification by flash chromatography (20:1 heptane:-ethyl acetate) affords 10.6 grams of the title compound as a white solid melting at 106-108°C. In contrast to the compound prepared in Example 3, the title compound consists of only one octanediyl isomer.

### Analysis:

- Calcd for C₄₀H₆₀N₂O₆:: C, 72.3; H, 9.1: N, 4.2.
- Found:: C, 72.4; H, 9.4; N, 4.0.

### Example 6A

### Bis(4-benzoyloxy-2,2,6,6-tetramethylpiperidin-1-yloxy)-heptane

The title compound, which consists of a mixture of heptanediyl isomers, is prepared according to the procedure of Example 3 by substitution of heptane for octane.

### Example 6B

### Bis(4-hydroxy-2,2,6,6-tetramethylpiperidin-1-yloxy)heptane

The title compound is prepared by basic hydrolysis (potassium hydroxide in ethanol) of the compound prepared in Example 6A.

### Example 7A

### Bis(4-benzoyloxy-2,2,6,6-tetramethylpiperidin-1-yloxy)-cyclohexane

The title compound, which consists of a mixture of cyclohexanediyl isomers, is prepared according to the procedure of Example 3 by substituting cyclohexane for octane.

### Example 7B

### Bis(4-hydroxy-2,2,6,6-tetramethylpiperidin-1-yloxy)cyclohexane

The title compound is prepared by basic hydrolysis (potassium hydroxide in ethanol) of the compound prepared in Example 7A.

### Example 8

### Bis[4-(3-dodecylsuccinimid-1-yl)-2,2,6,6-tetramethylpiperidin-1-yloxy]octane

The title compound, which consists of a mixture of octanedily isomers, is prepared according to the procedure of Example 3 by substituting 4-(3-dodecylsuccinimid-1-yl)-2,2,6,6-tetramethylpiperidine for 4-benzoyloxy-2,2,6,6-tetramethylpiperidine.

### Example 9

### Octanediylbis[N-[2-(ethoxycarbonylmethoxy)phenyl]-N'-(1-oxy-2,2,6,6-tetramethylpiperidin-4-yl)oxamide]

The title compound, which is a mixture of octanediyl isomers, is prepared according to the procedure of Example 3 by substituting N-[2-(ethoxycarbonylmethoxy)phenyl]-N'-(2,2,6,6-tetramethylpiperidin-4-yl)oxamide for 4-benzoyloxy-2,2,6,6-tetramethylpiperidine.

### Example 10A

### Bis(4-acetamido-2,2,6,6-tetramethylpiperidin-1-yloxy)octane

The title compound, which consists of a mixture of octanediyl isomers, is prepared by substituting 4-acetamido-2,2,6,6-tetramethylpiperidine for 4-benzoyloxy-2,2,6,6-tetramethylpiperidine in the procedure according to Example 3.

### Example 10B

### Bis(4-amino-2,2,6,6-tetramethylpiperidin-1-yloxy)octane

The title compound is prepared by the acidic hydrolysis (3N hydrochloric acid at reflux) of the compound prepared in Example 10A.

### Example 11

### Octanediylbis(methyl 1-oxy-2,2,6,6-tetramethylpiperidin-4-yl sebacate)

The title compound, which consists of a mixture of octanedily isomers, is prepared by substituting methyl 2,2,6,6-tetramethylpiperidin-4-yl sebacate for 4-benzoyloxy-2,2,6,6-tetramethylpiperidine in the procedure according to Example 3.

### Example 12

### Mixture of Bis- and Tris-(4-benzoyloxy-2,2,6,6-tetramethylpiperidin-1-yloxy)octadecane

A mixture of 80 mmol of 4-benzoyloxy-1-oxyl-2,2,6,6-tetramethylpiperidine, 250 mmol of 90% tert-butyl hydroperoxide, 125 grams of octadecane and 5 mmol of molybdenum trioxide is heated at 140°C in a Fischer-Porter prerssure bottle till the red color of the N-oxyl compound is no longer visible. The reaction mixture is purified by flash chromatography.

### Example 13

### 1,4-Bis(4-benzoyloxy-2,2,6,6-tetramethylpiperidin-1-yloxy)-1,2,3,4-tetrahydronaphthalene

A mixture of 80 mmol of 4-benzoyloxy-1-oxyl-2,2,6,6-tetramethylpiperidine, 300 mmol of 90% tert-butyl hydroperoxide, 5 mmol of molybdenum trioxide and 80 ml of 1,2,3,4-tetrahydronaphthalene (tetralin) is heated at 135°C in a Fischer-Porter pressure bottle till the red color of the N-oxyl starting material is no longer visible. Purification of the crude reaction mixture by flash chromatography affords the title compound.

### Example 14

### 1,4-Bis(4-octadecanoyloxy-2,2,6,6-tetramethylpiperidin-1-yloxy)butane

The title compound is prepared by reaction of a tetrahydrofuran solution of 1-hydroxy-4-octadecanoyloxy-2,2,6,6-tetramethylpiperidine with sodium hydride followed by reaction with 0.5 molar equivalent of 1,4-dibromobutane.

### Example 15

### 1,10-Bis(4-benzyloxy-2,2,6,6-tetramethylpiperidin-1-yloxy)decane

The title compound is prepared from 4-benzyloxy-1-oxyl-2,2,6,6-tetramethylpiperidin and 1,10-diiododecane according to the procedure of Example 5.

### Example 16

### Bis(4-benzoyloxy-2,2,6,6-tetramethylpiperidin-1-yloxy)methane

The reaction is carried out in a nitrogen atmosphere. Tributyltin hydride (20.0 grams, 68.7 mmol) is added dropwise over 2.75 hours to a solution, precooled to 10°C, of 40.0 grams (145 mmol) of 4-benzoyloxy-1-oxyl-2,2,6,6-tetramethylpiperidine, 9.0 grams (33.6 mmol) of methylene iodide (diiodomethane) and 75 ml of chlorobenzene. The reaction temperature reaches 27°C during the addition. The red mixture is stirred at room temperature for 27 hours after the addition is complete. The reaction mixture is then passed through a column of silica gel (heptane, then 100:3 heptane:ethyl acetate). Fractions containing the desired product are concentrated to give a crude solid. Tributyltin iodide is removed by washing a solution of the crude solid with aqueous ammonia. Final purification by flash chromatography on silica gel (100:3 heptane:ethyl acetate) followed by recrystallization from heptane affords 4.8 grams of the title compound as a white solid melting at 126-127°C.

### Analysis:

- Calcd for C₃₃H₄₆N₂O₆:: C, 69.9; H, 8.2; N, 4.9.
- Found:: C, 70.0; H, 8.2; N, 5.0.

### Example 17A

### Bis(4-acetamido-2,2,6,6-tetramethylpiperidin-1-yloxy)cyclooctane

The title compound, which consists of a mixture of cyclooctanediyl isomers, is prepared from 4-acetamido-2,2,6,6-tetramethylpiperidine and cyclooctane according to the procedure of Example 10A.

### Example 17B

### Bis(4-amino-2,2,6,6-tetramethylpiperidin-1-yloxy)cyclooctane

The title compound is prepared by the hydrolysis in 3N hydrochloric acid at reflux of the compound prepared in Example 17A.

### Example 18

### Mixture of Bis- and Tris(4-benzoyloxy-2,2,6,6-tetramethylpiperidin-1-yloxy)-decahydronaphthalene

The title mixture is prepared according to the procedure of Example 12 by substituting decahydronaphthalene (decalin) for octadecane.

### Example 19

### Bis(4-benzoyloxy-2,2,6,6-tetramethylpiperidin-1-yloxy)-methylcyclohexane

The title compound, which consists of a mixture of methylcyclohexanediyl isomers, is prepared from 4-benzoyloxy-1-oxyl-2,2,6,6-tetramethylpiperidine and methylcyclohexane according to the procedure of Example 3.

### Example 20

### Bis(4-acryloyloxy-2,2,6,6-tetramethylpiperidin-1-yloxy)-octane

The title compound is prepared by the reaction of the compound prepared in Example 4 with acryloyl chloride.

### Example 21

### 2,2-Bis(4-benzoyloxy-2,2,6,6-tetramethylpiperidin-1-oxy)-propane

The title compound is prepared from 4-benzoyloxy-1-oxyl-2,2,6,6-tetramethylpiperidine and 2,2-dibromopropane according to the procedure of Example 16.

### Example 22

### Cyclohexanediyl Bis [(1-cyclohexyloxy-2,2,6,6-tetramethylpiperidin-4-yl) (1-oxy-2,2,6,6-tetramethylpiperidin-4-yl) sebacate]

A mixture of 20.0 g (41.6 mmol) of di-(2,2,6,6-tetramethylpiperidin-4-yl) sebacate, 43 g (334 mmol) of 70% aqueous t-butyl hydroperoxide, 1.3 g (9.0 mmol) of molybdenum trioxide, and 125 ml of cyclohexane is heated at reflux for 2.3 hours. Water is collected in a Dean-Stark trap. The red reaction mixture is cooled and transferred to a Fischer-Porter bottle. Fresh cyclohexane (25 ml) is used to thoroughly rinse the flask, and the rinsings are added to the pressure bottle. The pressure bottle is immersed in an oil bath (140°C) for 3 hours whereupon the colorless reaction mixture is cooled to room temperature and filtered. The filtrate is stirred with 10 g of sodium sulfite in 90 ml of water for 2 hours to decompose unreacted hydroperoxide, then diluted with ethyl acetate (200 ml) and water (100 ml). The organic layer is washed with 10% sodium sulfite (100 ml), water (100 ml), saturated sodium chloride (100 ml), then dried over magnesium sulfate and concentrated at reduced pressure. The crude product is purified by flash chromatography (silica gel, 50:1 heptane:ethyl acetate) to afford 17.8 g (63% yield) of a white solid, m.p. 56-9°C, which is di-(1-cyclohexyloxy-2,2,6,6-tetramethylpiperidin-4-yl) sebacate. The column is then eluted with 10:1 heptane:ethyl acetate to obtain the title compound as a mixture of cyclohexanediyl isomers.

### Example 23

### Heptanediyl Bis [(1-heptyloxy-2,2,6,6-tetramethylpiperidin-4-yl) (1-oxy-2,2,6,6-tetramethylpiperidin-4-yl) sebacate]

A mixture of 35.0 g (72.8 mmol) of di-(2,2,6,6-tetra-methylpiperidin-4-yl) sebacate, 58.3 (582 mmol) of 90% aqueous t-butyl hydroperoxide, 2.0 g of molybdenum trioxide, and 250 ml of heptane is heated at 140°C in a Fischer-Porter bottle. The pressure is maintained at 40-50 psi by occasional venting. Heating is discontinued after 7 hours. An additional portion (20.0 g) of 90% t-butyl hydroperoxide is added and the reaction mixture is heated for one hour at 140°C. The reaction is nearly colorless by this time. The reaction mixture is cooled and filtered to remove the catalyst. The organic phase is separated, dried over magnesium sulfate, and concentrated to 100 ml total volume. This solution is passed through silica gel with heptane as the eluent. The filtrate is evaporated to yield 36.9 g (72% yield) of di-(1-heptyloxy-2,2,6,6-tetramethylpiperidin-4-yl) sebacate, a nearly colorless oil. Elution of the column with 10:1 heptane:ethyl acetate then affords the title compound as a mixture of heptanediyl isomers.

### Example 24

### Octanediyl Bis[(1-octyloxy-2,2,6,6-tetramethylpiperidin-4-yl) (1-oxy-2,2,6,6-tetramethylpiperidin-4-yl) sebacate]

70% Aqueous t-butyl hydroperoxide (140 g, 1.09 mol) is added over a 6 hour period to a mixture of 75.4 g (0.157 mol) of bis(2,2,6,6-tetramethylpiperidin-4-yl) sebacate, 1.25 g (8.7 mmol) of molybdenum trioxide, and 570 ml of n-octane that has been heated to 115°C under a nitrogen atmosphere. During the addition, the reaction is maintained at reflux. Water is collected in a Dean-Stark trap. Upon completion of the addition, the red reaction mixture is heated at reflux (95-97°C) for seven hours to discharge the red color. The molybdenum trioxide is removed by filtration. The yellow filtrate is stirred at ambient temperature for 30 minutes with 15 g of activated charcoal (DARCO) to remove some of the yellow color, and then concentrated at reduced pressure. The crude product is purified by flash chromatography on silica gel (100:3 heptane: ethyl acetate) to afford 92.9 g (80% yield) of di(1-octyloxy-2,2,6,6-tetramethylpiperidin-4-yl) sebacate, a colorless oil. The column is then eluted with 10:1 heptane:ethyl acetate to obtain the title compound as a mixture of octandiyl isomers. The title compound is a colorless oil.

### Analysis:

- Calcd. for C₈₀H₁₅₀N₄O₁₂:: C, 70.7; H, 11.1; N, 4.1.
- Found:: C, 69.8; H, 11.5; N, 4.1.

### Example 25

### Cyclohexanediyl Bis[(1-cyclohexyloxy-2,2,6,6-tetramethylpiperidinyl-4-yl) (1-oxy-2,2,6,6-tetramethylpiperidin-4-yl) succinate]

A two-phase mixture of 70% aqueous tert-butyl hydroperoxide (103.9 grams, 807 mmol), cyclohexane (200 ml) and sodium chloride (15 grams) is shaken in a separatory funnel. The organic phase is dried over anhydrous magnesium sulfate, filtered and added to 40 grms (101 mmol) of bis(2,2,6,6-tetramethylpiperidin-4-yl) succinate. Molybdenum trioxide (2.0 grams) is added and the mixture is heated at reflux for one hour. Water is collected in a Dean-Stark trap. The entire reaction mixture is then transferred to a Fischer-Porter pressure bottle and heated for six hours at 140°C. Additional tert-butyl hydroperoxide (90%, 10.1 grams, 101 mmol) is added and heating is resumed for another four hours. The colorless reaction mixture is filtered, concentrated and dissolved in heptane (200 ml). The heptane solution is passed through a column of silica gel with heptane as the eluent to obtrain 41.2 grams (69% yield) of bis(1-cyclohexyloxy-2,2,6,6-tetramethylpiperidin-4-yl) succinate as a white solid melting at 122-126°C. Further elution of the column with 10:1 heptane:ethyl acetate affords the title compound as a mixture of cyclohexanediyl isomers.

### Example 26

### Oligomers of Bis(1-oxy-2,2,6,6-tetramethylpiperidin-4-yl) Sebacate and Octanediyl

When the procedure of Example 24 is repeated using 200 ml of n-octane, a mixture of oligomers containing 2 to 5 bis(1-oxy-2,2,6,6-tetramethylpiperidin-4-yl) sebacate moieties is obtained.

### Example 27

### Oligomers of Bis(1-oxy-2,2,6,6-tetramethylpiperidin-4-yl) Sebacate and Heptanediyl

When the procedure of Example 23 is repeated using 80 ml of heptane, a mixture of oligomers containing 2 to 5 bis(1-oxy-2,2,6,6-tetramethylpiperidin-4-yl) sebacate moieties is obtained.

### Example 28

### Oligomers of Bis(1-oxy-2,2,6,6-tetramethylpiperidin-4-yl) sebacate and Cyclohexanediyl

When the procedure of Example 22 is repeated using 40 ml of cyclohexane, a mixture of oligomers containing 2 to 5 bis(1-oxy-2,2,6,6-tetramethylpiperidin-4-yl) sebacate moieties is obtained.

### Example 29

### Oligomers of Bis(1-oxy-2,2,6,6-tetramethylpiperidin-4-yl Succinate and Cyclohexanediyl

When the procedure of Example 25 is repeated using 75 ml of cyclohexane, a mixture of oligomers containing 2 to 5 bis(1-oxy-2,2,6,6-tetramethylpiperidin-4-yl) succinate moieties is obtained.

### Example 30

### Cyclohexanediyl Bis[N,N'-dibutyl-N-(1-cyclohexyloxy-2,2,6,6-tetramethylpiperidin-4-yl)-N'-(1-oxy-2,2,6,6-tetra methyl-piperidin-4-yl)oxamide]

The title compound is prepared by substituting N,N'dibutyl-N,N'-di(2,2,6,6-tetramethylpiperidin-4-yl)oxamide for bis(2,2,6,6-tetramethylpiperidin-4-yl) succinate in the procedure of Example 25.

### Example 31

### Oligomers of Bis[2-((1-oxy-2,2,6,6-tetramethylpiperidin-4-yl)-aminocarbonyl)methoxy]oxanilide and Octanediyl

A mixture of oligomers is prepared by substituting bis[2-((2,2,6,6-tetramethylpiperidin-4-yl)aminocarbonyl)methoxy]oxanilide for bis(2,2,6,6-tetramethylpiperidin-4-yl) sebacate in the procedure of Example 26.

### Example 32

### Oligomers of 3,15-Diylbis(oxy)-2,2,4,4,14,14,16,16-octamethyl-7,11,18,21-tetraoxa-3,15-diazatrispiro[5.2.2.5.2]-heneicosane and 1,4-Butanediyl

Excess sodium hydride is added to a solution of 100 mmol of 3,15-diyl-bis(hydroxy)-2,2,4,4,14,14,16,16-octamethyl-7,11,18,21-tetraoxa-3,15-diazatrispiro[5.2.2.5.2.2.]-heneicosane in tetrahydrofuran. To this solution is added dropwise a solution of 125 mol of 1,4-diiodobutane in tetrahydrofuran. After the alkylation reaction is complete, the reaction mixture is treated with tributyltin hydride and a catalytic amount of 2,2'-azobis(2-methyl-propionitrile) to reduce the terminal N-(4-iodobutyloxy) residues.

### Example 33

### Oligomers of Bis(1-oxy-2,2,6,6-tetramethylpiperidin-4-yl) Isophthalate and 1,8-octanediyl

Tributyltin hydride (200 mmol) is added dropwise to a solution of 175 mmol of bis(1-oxyl-2,2,6,6-tetramethylpiperidin-4-yl) isophthalate and 100 mmol of 1,8-diiodo-octane in chlorobenzene. The crude reaction mixture is passed through a column of silica gel with hexane as the eluent to remove solvent. Further elution with hexane:ethyl acetate affords the product as a mixture of oligomers.

### Example 34A

### 1-Octyloxy-2,2,6,6-tetramethylpiperidin-4-yl 1-Oxy-2,2,6,6-tetramethylpiperidin-4-yl Sebacate

Aqueous tert-butyl hydroperoxide (70%, 80.0 grams, 0.621 mol) is added over a five-hour period to a mixture that is preheated to 125°C of 60.4 grams (0.125 mol) of bis(2,2,6,6-tetramethylpiperidin-4-yl) sebacate, 1.8 grams of molybdenum trioxide and 620 ml of n-octane under a nitrogen atmosphere. During the addition, the reaction mixture is maintained at reflux. Water is collected in a Dean-Stark trap. The red reaction mixture is heated at reflux for two hours after the addition is complete. Solids are removed by filtration, and the red filtrate is concentrated at reduced pressure. The concentrate is purified by flash chromatography on silica gel (20:1, then 10:1 heptane:ethyl acetate) to afford 26.4 grams of the title compound as a red oil.

### Example 34B

### 1,8-Octanediylbis[(1-octyloxy-2,2,6,6-tetramethylpiperidin-4-yl) (1-oxy-2,2,6,6-tetramethylpiperidin-4-yl) sebacate]

A solution of 26.4 grams (42.1 mmol) of the compound formed in Example 34A, 3.6 grams (9.8 mmol) of 1,8-diiodooctane and 50 ml of chlorobenzene is cooled to 10°C under a nitrogen atmosphere. Tributyltin hydride (7.0 grams, 24.1 mmol) is added dropwise over a 45-minute period to the chilled solution. The reaction mixture is then stirred at room temperature for 16 hours and finally poured onto a column of silica gel. The column is successively eluted with heptane, 100:3 heptane:ethyl acetate, and 50:3 heptane:ethyl acetate. Fractions containing the crude product are combined and evaporated to yield an oil which is washed with aqueous ammonia to remove tributyltin iodide. Final purification by flash chromatography on silica gel (50:3 heptane:ethyl acetate) affords 3.0 grams of the title compound as a colorless oil.

### Analysis:

- Calcd. for C₈₀H₁₅₀N₄O₁₂:: C, 70.7; H, 11.1; N, 4.1.
- Found:: C, 71.0; H, 11.7; N, 3.9.

### Example 35A

### 4-Hydroxy-1-octyloxy-2,2,6,6-tetramethylpiperidine

The title compound is prepared by the dropwise addition of tributyltin hydride to a solution of 4-hydroxy-1-oxyl-2,2,6,6-tetramethylpiperidine and 1-iodooctane in chlorobenzene under a nitrogen atmosphere.

### Example 35B

### 1-octyl-2,2,6,6-tetramethylpiperidin-4-yl 1-Oxyl-2,2,6,6-tetramethylpiperidin-4-yl Isophthalate

Isophthaloyl dichloride is added dropwise to a mixture of the compound prepared in Example 35A, 4-hydroxy-1-oxyl-2,2,6,6-tetramethylpiperidin, triethylamine and methylene chloride. The reaction mixture is worked up in the usual manner and purified by flash chromatography on silica gel to afford the title compound.

### Example 35C

### 1,8-Octanediylbis[(1-octyloxy-2,2,6,6-tetramethylpiperidin-4-yl) (1-oxy-2,2,6,6-tetramethylpiperidin-4-yl) isophthalate]

Tributyltin hydride is added to a chlorobenzene solution of 1,8-diiodooctane and the compound prepared in Example 14B. Purification by flash chromatography affords 1-(8-iodooctyloxy)-2,2,6,6-tetramethylpiperidin-4-yl 1-octyloxy-2,2,6,6-tetramethylpiperidin-4-yl isophthalate and the title compound.

The structure of the title compound of Example 35C is seen by reference to the formula A 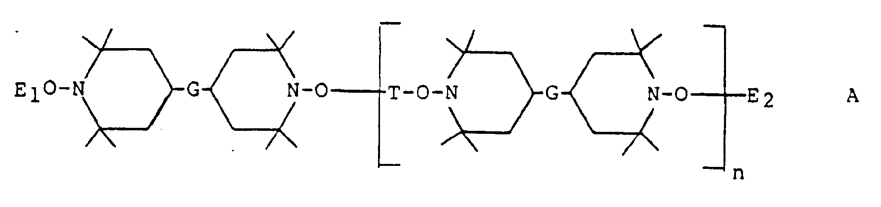
wherein n is 1; G is isophthalate; D is 1,8-octanediyl and E₁ and E₂ are both octyl.

### Example 36A

### (1-[8-(4-Hydroxy-2,2,6,6-tetramethylpiperidin-1-yloxy)]-octyloxy-2,2,6,6-tetramethylpiperidin-4-yl) (1-Octyloxy-2,2,6,6-tetramethylpiperidin-4-yl) Isophthalate

The title compound is prepared by the addition of tributyltin hydride to a chlorobenzene solution containing 4-hydroxy-1-oxyl-2,2,6,6-tetramethylpiperidin and 1-(8-iodo-octyloxy)-2,2,6,6-tetramethylpiperidin-4-yl 1-octyloxy-2,2,6,6-tetramethylpiperidin-4-yl isophthalate isolated in Example 35C.

### Example 36B

### Compound of Formula A where n is 2, G is isophthalate, T is octanediyl and E₁ and E₂ are each octyl

The title compound is prepared by reaction of isophthaloyl dichloride with a mixture of triethylamine, methylene chloride and the compound prepared in Example 36A.

### Example 37A

### 1-Methoxy-2,2,6,6-tetramethylpiperidin-4-yl 1-Oxyl-2,2,6,6-tetramethylpiperidin-4-yl Sebacate

Sebacoyl chloride is added to a mixture of 4-hydroxy-1-methoxy-2,2,6,6-tetramethylpiperidin, 4-hydroxyl-1-oxyl-2,2,6,6-tetramethylpiperidin, triethylamine and methylene chloride. The reaction mixture is worked up in the usual manner and purified by flash chromatography on silica gel to afford the title compound.

### Example 37B

### Methylenebis(1-methoxy-2,2,6,6-tetramethylpiperidin-4-yl 1-oxo-2,2,6,6-tetramethylpiperidin-4-yl sebacate)

The title compound is prepared by the addition of tributyltin hydride to a chlorobenzene solution of diiodo-methane and the compound prepared in Example 37A.

### Example 38A

### Compound of Formula A where n is 1, G is isophthalate, T is octanediyl, E₁ is octyl and E₂ is oxyl

Isophthaloyl dichloride is added to a mixture of 4-hydroxy-1-oxyl-2,2,6,6-tetramethylpiperidine, the compound prepared in Example 36A, triethylamine and methylene chloride. The reaction mixture is worked up in the usual manner and purified by flash chromatography on silica gel to afford the title compound.

### Example 38B

### Compound of Formula A where n is 3, G is isophthalate, T is octanediyl and E₁ and E₂ are both octyl

Tributyltin hydride is added to a chlorobenzene solution of 1,8-diiodooctane and N-oxyl compound prepared in Example 36A. Purification by flash chromatography affords the title compound.

A byproduct isolated from the reaction mixture is the compound of formula A where n is 1, G is isophthalate, T is octanediyl, E₁ is octyl and E₂ is -(CH₂)₇CH₂I.

### Example 39

### Compound of Formula A where n is 4, G is isophthalate, T is octanediyl and E₁ and E₂ are both octyl

The title compound is prepared by the reaction of bis(1-hydroxy-2,2,6,6-tetramethylpiperidin-4-yl) isophthalate with sodium hydride followed by reaction with the byproduct isolated in Example 38B which is the compound of formula A where n is 1, G is isophthalate, T is octanediyl, E₁ is octyl and E₂ is -(CH₂)₇CH₂I.

### Example 4O

### Light Stabilization of Polypropylene

This example illustrates the light stabilizing effectiveness of instant stabilizers.

Unstabilized polypropylene powder (Hercules Profax 6501) is throughly blended with O.2% by weight of a compound of formula I or II. The blended materials are then milled on a two-roll mill at 182°C for five minutes, after which time the stabilized polypropylene is sheeted from the mill and allowed to cool. The milled polypropylene is then cut into pieces and compression molded on a hydraulic press at 220°C and 1.2 x 1O⁶ Pa into O.127 mm films. The sample is exposed in a fluorescent sunlight/black light chamber until failure. Failure is taken as the hours required to reach 0.5 carbonyl absorbance by infrared spectroscopy on the exposed films.

Each of the compounds of Examples 1,2 and 3 are effective as light stabilizers for protecting polypropylene from the deleterious effects of actinic light.

### Example 41

### Stabilization of High Solids Thermoset Acrylic Resin Enamel

A thermoset acrylic enamel based on a binder of 70% by weight of a copolymer of 2-hydroxyethyl acrylate, butyl acrylate, methyl methacrylate, styrene and acrylic acid and of 3O% by weight of a melamine resin in the presence of an acid catalyst, p-toluenesulfonic acid, dinonylnaphthalene disulfonic acid or dodecylbenzenesulfonic acid, is formulated to include 2% by weight based on the resin solids of a benzotriazole ultraviolet absorber and an effective stabilizing amount of the test hindered amine light stabilizer.

Commercially available epoxy primed 1O.16 cm x 3O.48 cm panels (Uniprime from Advanced Coatings Technology) are spray coated with a silver metallic basecoat to a thickness of about O.O23 mm and air dried for 3 minutes. The stabilized thermoset acrylic resin enamel is then sprayed onto the basecoated panel to a thickness of about O.O49 mm. After 15 minutes air-drying, the coated sheets are baked for 3O minutes at 121°C.

After storage for 1 week in a air-conditioned room, the coated panels are subjected to weathering in a QUV exposure apparatus according to test method ASTM G-53/77. In this test, the samples are subjected to weathering in repeated cycles for 4 hours in a humid atmosphere at 50°C and then for 8 hours under UV light at 70°C. The panels are exposed in the QUV for 1500 hours. The 20° gloss values of the panels are determined before and after exposure.

The loss of gloss of the stabilized panels is considerably less than that of the unstabilized control panels.

## Claims

1. A poly (1-hydrocarbyloxy-2,2,6,6-tetramethyl-piperidine) compound of the formula I or II 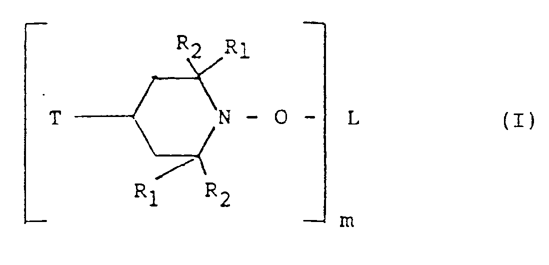 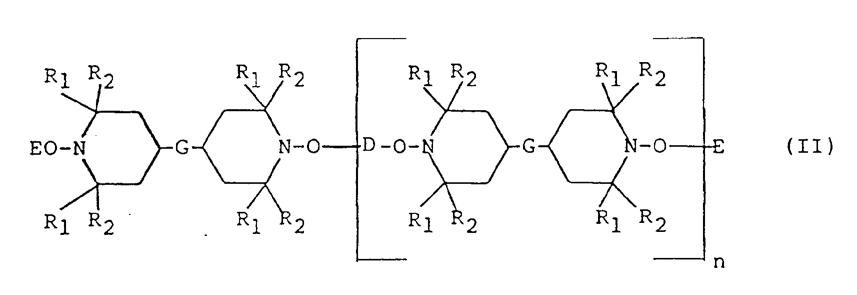 where
m is 2 to 10, n is 1 to 5,
R₁ and R₂ are independently alkyl of 1 to 4 carbon atoms, or R₁ and R₂ together are pentamethylene,
L is an m-valent radical of an alkane of 1 to 18 carbon atoms or of an alkene of up to 18 carbon atoms, an m-valent radical of a cycloalkane or cycloalkene of 5 to 12 carbon atoms, an m-valent radical of a saturated or unsaturated bicyclic or tricyclic hydrocarbon of 7 to 12 carbon atoms or an m-valent radical of an aryl, alkyl substituted aryl or aralkyl hydrocarbon of 6 to 15 carbon atoms, with the proviso that the N-O groups are not necessarily attached to the same carbon atom in L,
T is an organic moiety selected from the group consisting of R₃-COO-, R₄-OCO-, R₃-CONR₅-, 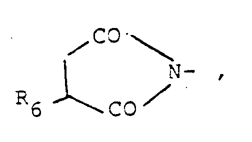 R₄NHCOCONH-, 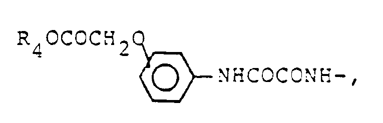 R₄OCO(CH₂)_{q}NH-, R₄OCO(CH₂)ₚCOO-, 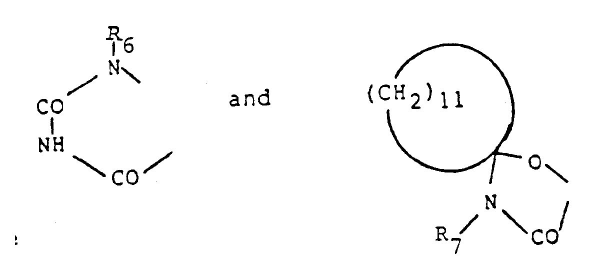 where
R₃ is a radical of an aliphatic, cycloaliphatic, unsaturated aliphatic or aromatic carboxylic acid without the carboxy group,
R₄ is a radical of an aliphatic, cycloaliphatic or unsaturated aliphatic alcohol without the hydroxy group,
R₅ is hydrogen, alkyl of 1 to 8 carbon atoms or 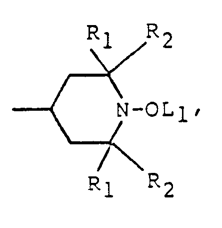 where L₁ is a monovalent radical of the definition of L,
p is O to 1O, q is 2 to 4,
R₆ is hydrogen or alkyl of 1 to 18 carbon atoms,
R₇ is hydrogen or -CH₂CH₂COOC₁₂H₂₅,
D is a diradical of an alkane or alkene of 1 to 18 carbon atoms, diradical of a cycloalkane or cycloalkene of 5 to 12 carbon atoms, or a diradical of a saturated or unsaturated bicyclic or tricyclic hydrocarbon of 7 to 12 carbon atoms, with the proviso that the N-O groups are not necessarily attached to the same carbon atom in D,
E is a monovalent radical of an alkane or alkene of 1 to 18 carbon atoms, a monovalent radical of a cycloalkane or cycloalkene of 5 to 12 carbon atoms or a monovalent radical of a saturated or unsaturated bicyclic or tricyclic hydrocarbon of 7 to 12 carbon atoms,
G is an organic linking moiety selected from the group consisting of -OCO-R₈-COO-, -COO-R₉-OCO-, -NR₅-CO-R₈-CONR₅-, 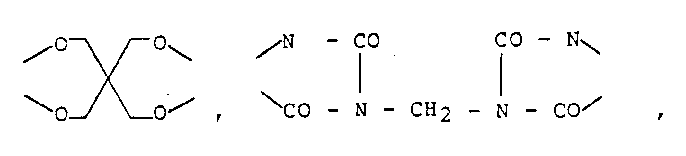 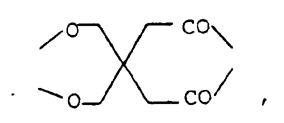 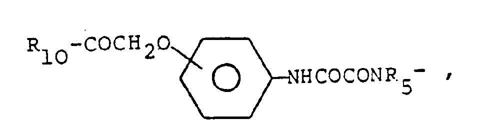 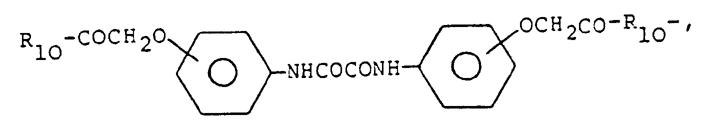 and 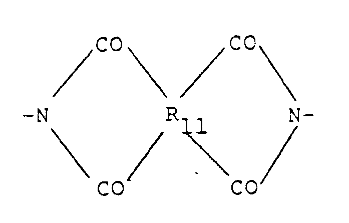 where
R₈ is a direct bond or a divalent radical of an aliphatic, cycloaliphatic, unsaturated aliphatic or aromatic dicarboxylic acid without the two carboxy groups,
R₉ is a divalent radical of an aliphatic, cycloaliphatic or unsaturated aliphatic diol without the two hydroxy groups,
R_{1O} is -O- or -NR₅-, and
R₁₁ is an aliphatic or aromatic tetravalent radical.

2. A compound of formula I according to claim 1 wherein m is 2 to 4.

3. A compound according to claim 2 wherein m is 2.

4. A compound of formula II according to claim 1 wherein n is 1 or 2.

5. A compound of formula I or II according to claim 1 wherein R₁ and R₂ are each methyl.

6. A compound of formula I according to claim 1 wherein L is an m-valent radical of n-octane, n-heptane or cyclohexane.

7. A compound of formula I according to claim 1 wherein T is R₃-COO- where R₃ is phenyl, vinyl or alkyl of 1 to 17 carbon atoms.

8. A compound according to claim 7 wherein R₃ is heptadecyl.

9. A compound of formula I according to claim 1 wherein T is R₃-CONR₅- where R₅ is hydrogen and R₃ is vinyl.

10. A compound of formula I according to claim 1 wherein T is 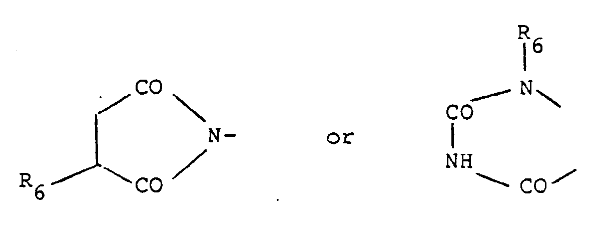 where R₆ is dodecyl.

11. A compound of formula II according to claim 1 wherein D is a diradical of n-octane, n-heptane or cyclohexane.

12. A compound of formula II according to claim 1 wherein E is octyl, heptyl or cyclohexyl.

13. A compound of formula II according to claim 1 wherein G is -OCO-R₈-COO- where R₈ is alkylene of 2 to 8 carbon atoms or phenylene.

14. A compound according to claim 13 wherein R₈ is ethylene or octamethylene.

15. A compound of formula II according to claim 1 wherein G is -NR₅-CO-R₈-CO-NR₅- where R₅ is hydrogen or butyl and R₈ is a direct bond or alkylene of 2 to 8 carbon atoms.

16. A compound according to claim 15 wherein R₈ is a direct bond, ethylene or octamethylene.

17. A compound of formula II according to claim 1 where G is

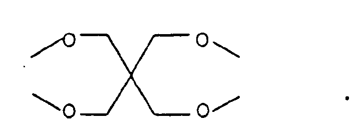

18. The compound according to claim 1 which is 1,4-bis(4-benzoyloxy-2,2,6,6-tetramethylpiperidin-1-yloxy)-2-cyclohexene.

19. The compound according to claim 1 which is 1,8-bis-(4-benzoyloxy-2,2,6,6-tetramethylpiperidine-1-yl-oxy)octane.

20. The compound according to claim 1 which is bis-(4-benzoyloxy-2,2,6,6-tetramethylpiperidin-1-yloxy)octane.

21. The compound according to claim 1 which is bis-(4-benzoyloxy-2,2,6,6-tetramethylpiperidin-1-yloxy)methane,

22. A compound according to claim 1 which is cyclohexanediyl bis[(1-cyclohexyloxy-2,2,6,6-tetramethylpiperidin-4-yl) (1-oxy-2,2,6,6-tetramethylpiperidin-4-yl) sebacate].

23. The compound according to claim 1 which is heptanediyl bis[(1-heptyloxy-2,2,6,6-tetramethylpiperidin-4-yl) (1-oxy-2,2,6,6-tetramethylpiperidin-4-yl) sebacate].

24. The compound according to claim 1 which is octanediyl bis[(1-octyloxy-2,2,6,6-tetramethylpiperidin-4-yl) (1-oxy-2,2,6,6-tetramethylpiperidin-4-yl) sebacate].

25. The compound according to claim 1 which is 1,8-octanediyl bis[(1-octyloxy-2,2,6,6-tetramethylpiperidin-4-yl) (1-oxy-2,2,6,6-tetramethylpiperidin-4-yl) sebacate].

26. A stabilized organic polymer composition which comprises
(a) an organic polymer subject to deterioration by the adverse effects of actinic light, and
(b) a stabilizing amount of a compound of formula I or II according to claim 1.

27. A composition according to claim 26 wherein the organic polymer is a polyolefin.

28. A composition according to claim 27 wherein the polyolefin is polypropylene.

29. A composition according to claim 26 wherein the component (b) is 1,8-bis(4-benzoyloxy-2,2,6,6-tetramethylpiperidine-1-oxy)octane.

30. A composition according to claim 26 wherein the organic polymer is a coating system based on alkyd, acrylic, acrylic alkyd, polyester, epoxide, urethane, polyamide, vinyl or epoxy-polyester resins.

31. A composition according to claim 30 which contains a UV absorber or additional light stabilizer.

32. A method for stabilizing an organic material against oxidative, thermal or actinic degradation which comprises incorporating into said organic material an effective stabilizing amount of a compound of formula I or II according to claim 1.

## Patentansprüche

1. Poly(1-hydrocarbyloxy-2,2,6,6-tetramethylpiperidin) verbindung der Formel I oder II 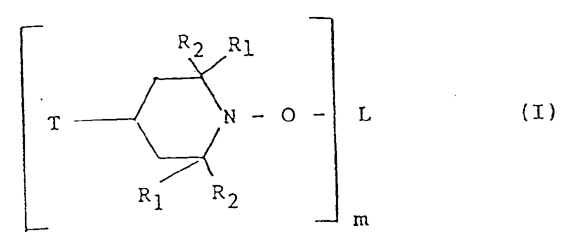 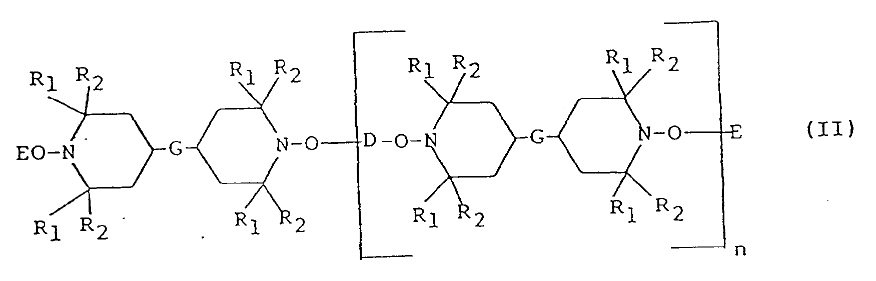 worin
m den Wert 2 bis 10 hat, n den Wert 1 bis 5 hat,
R₁ und R₂ unabhängig voneinander einen Alkylrest mit 1 bis Kohlenstoffatomen bedeuten, oder R₁ und R₂ zusammen Pentamethylen bedeuten,
L ein m-wertiger Rest eines Alkans mit 1 bis 18 Kohlenstoffatomen oder eines Alkens mit bis zu 18 Kohlenstoffatomen, ein m-wertiger Rest eines Cycloalkans oder Cycloalkens mit 5 bis 12 Kohlenstoffatomen, ein m-wertiger Rest eines gesättigten oder ungesättigten bicyclischen oder tricyclischen Kohlenwasserstoffs mit 7 bis 12 Kohlenstoffatomen oder ein m-wertiger Rest eines Aryl-, Alkylsubstituierten Aryl- oder Aralkylkohlenwasserstoffs mit 6 bis 15 Kohlenstoffatomen ist, mit der Maßgabe, daß die N-O-Gruppen nicht notwendigerweise an das gleiche Kohlenstoffatom in L gebunden sind,
T eine organische Gruppierung, ausgewählt aus der Gruppe bestehend aus R₃-COO-, R₄-OCO-, R₃-CONR₅-, 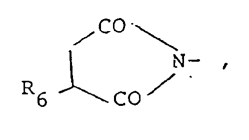 R₄NHCOCONH-, 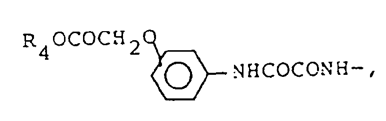 R₄OCO(CH₂)_{q}NH-, R₄OCO(CH₂)ₚCOO-, 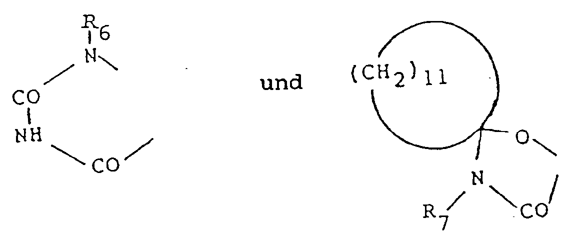 worin
R₃ ein aliphatischer, cycloaliphatischer, ungesättigter aliphatischer oder aromatischer Carbonsäurerest ohne die Carboxylgruppe ist,
R₄ ein aliphatischer, cycloaliphatischer oder ungesättigter aliphatischer Alkoholrest ohne die Hydroxylgruppe ist,
R₅ ein Wasserstoffatom, ein Alkylrest mit 1 bis 8 Kohlenstoffatomen oder 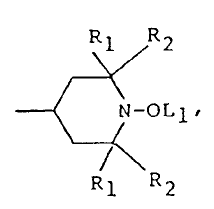 ist, worin L₁ ein einwertiger Rest der Definition von L ist,
p den Wert 0 bis 10 hat, q den Wert 2 bis 4 hat,
R₆ ein Wasserstoffatom oder ein Alkylrest mit 1 bis 18 Kohlenstoffatomen ist,
R₇ ein Wasserstoffatom oder -CH₂CH₂COOC₁₂H₂₅ ist,
D ein zweiwertiger Rest eines Alkans oder Alkens mit 1 bis 18 Kohlenstoffatomen, ein zweiwertiger Rest eines Cycloalkans oder Cycloalkens mit 5 bis 12 Kohlenstoffatomen oder ein zweiwertiger Rest eines gesättigten oder ungesättigten bicyclischen oder tricyclischen Kohlenwasserstoffs mit 7 bis 12 Kohlenstoffatomen ist, mit der Maßgabe, daß die N-O-Gruppen nicht notwendigerweise an das gleiche Kohlenstoffatom in D gebunden sind,
E ein einwertiger Rest eines Alkans oder Alkens mit 1 bis 18 Kohlenstoffatomen, ein einwertiger Rest eines Cycloalkans oder Cycloalkens mit 5 bis 12 Kohlenstoffatomen oder ein einwertiger Rest eines gesättigten oder ungesättigten bicyclischen oder tricyclischen Kohlenwasserstoffs mit 7 bis 12 Kohlenstoffatomen ist,
G eine organische Verbindungseinheit, ausgewählt aus der Gruppe bestehend aus -OCO-R₈-COO-, -COO-R₉-OCO-, -NR₅-CO-R₈-CONR₅-, 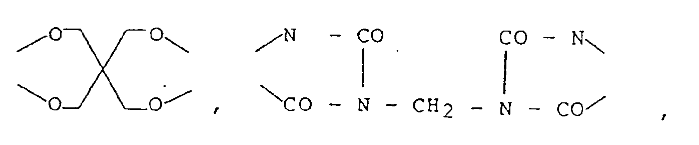 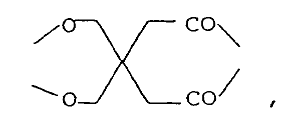 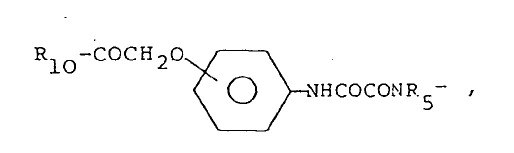 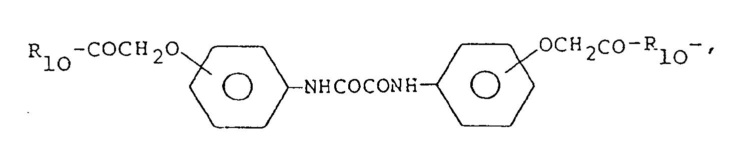 und 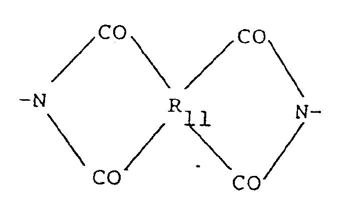 ist, worin
R₈ eine direkte Bindung oder ein zweiwertiger Rest einer aliphatischen, cycloaliphatischen, ungesättigten aliphatischen oder aromatischen Dicarbonsäure ohne die zwei Carboxylgruppen ist,
R₉ ein zweiwertiger Rest eines aliphatischen, cycloaliphatischen oder ungesättigten aliphatischen Diols ohne die zwei Hydroxylgruppen ist,
R₁₀ -O- oder -NR₅- ist, und
R₁₁ ein aliphatischer oder aromatischer vierwertiger Rest ist.

2. Verbindung der Formel I nach Anspruch 1, worin m den Wert 2 bis 4 besitzt.

3. Verbindung nach Anspruch 2, worin m den Wert 2 besitzt.

4. Verbindung der Formel II nach Anspruch 1, worin n den Wert 1 oder 2 besitzt.

5. Verbindung der Formel I oder II nach Anspruch 1, worin R₁ und R₂ jeweils Methyl bedeuten.

6. Verbindung der Formel I nach Anspruch 1, worin L ein m-wertiger n-Octan-, n-Heptan- oder Cyclohexanrest ist.

7. Verbindung der Formel I nach Anspruch 1, worin T für R₃-COO- steht, wobei R₃ Phenyl, Vinyl oder Alkyl mit 1 bis 17 Kohlenstoffatomen bedeutet.

8. Verbindung nach Anspruch 7, worin R₃ für Heptadecyl steht.

9. Verbindung der Formel I nach Anspruch 1, worin T für R₃-CONR₅- steht, wobei R₅ ein Wasserstoffatom bedeutet, und R₃ für Vinyl steht.

10. Verbindung der Formel I nach Anspruch 1, worin T für 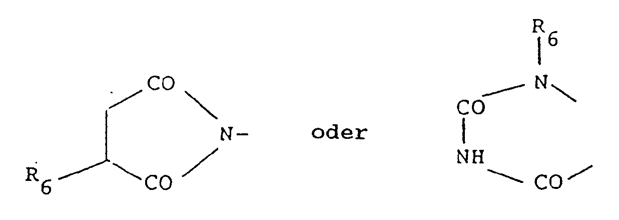 worin R₆ Dodecyl bedeutet, steht.

11. Verbindung der Formel II nach Anspruch 1, worin D ein zweiwertiger n-Octan-, n-Heptan- oder Cyclohexanrest ist.

12. Verbindung der Formel II nach Anspruch 1, worin E für Octyl, Heptyl oder Cyclohexyl steht.

13. Verbindung der Formel II nach Anspruch 1, worin G für -OCO-R₈-COO-, worin R₈ ein Alkylenrest mit 2 bis 8 Kohlenstoffatomen oder ein Phenylenrest ist, steht.

14. Verbindung nach Anspruch 13, worin R₈ Ethylen oder Octamethylen bedeutet.

15. Verbindung der Formel II nach Anspruch 1, worin G für -NR₅-CO-R₈-CO-NR₅-, worin R₅ ein Wasserstoffatom oder Butyl bedeutet, und R₈ eine direkte Bindung oder ein Alkylenrest mit 2 bis 8 Kohlenstoffatomen ist, steht.

16. Verbindung nach Anspruch 15, worin R₈ eine direkte Bindung, eine Ethylen- oder Octamethylengruppe ist.

17. Verbindung der Formel II nach Anspruch 1, worin G 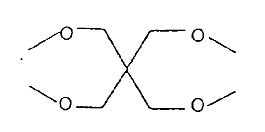 bedeutet.

18. Verbindung nach Anspruch 1, nämlich 1,4-Bis-(4-benzoyloxy-2,2,6,6-tetramethylpiperidin-1-yloxy)-2-cyclohexen.

19. Verbindung nach Anspruch 1, nämlich 1,8-Bis-(4-benzoyloxy-2,2,6,6-tetramethylpiperidin-1-yloxy)octan.

20. Verbindung nach Anspruch 1, nämlich Bis-(4-benzoyloxy-2,2,6,6-tetramethylpiperidin-1-yloxy)octan.

21. Verbindung nach Anspruch 1, nämlich Bis-(4-benzoyloxy-2,2,6,6-tetramethylpiperidin-1-yloxy)methan.

22. Verbindung nach Anspruch 1, nämlich Cyclohexandiyl-bis-[(1-cyclohexyloxy-2,2,6,6-tetramethylpiperidin-4-yl)(1-oxy-2,2,6,6-tetramethylpiperidin-4-yl)sebacat].

23. Verbindung nach Anspruch 1, nämlich Heptandiyl-bis-[(1-heptyloxy-2,2,6,6-tetramethylpiperidin-4-yl) (1-oxy-2,2,6,6-tetramethylpiperidin-4-yl)sebacat].

24. Verbindung nach Anspruch 1, nämlich Octandiyl-bis-[(1-octyloxy-2,2,6,6-tetramethylpiperidin-4-yl) (1-oxy-2,2,6,6-tetramethylpiperidin-4-yl)sebacat].

25. Verbindung nach Anspruch 1, nämlich 1,8-Octan-diyl-bis-[(1-octyloxy-2,2,6,6-tetramethylpiperidin-4-yl)-(1-oxy-2,2,6,6-tetramethylpiperidin-4-yl)sebacat].

26. Stabilisierte organische Polymermasse, umfassend
(a) ein organisches Polymeres, das durch die nachteiligen Wirkungen von actinischem Licht verschlechtert wird, und
(b) eine stabilisierende Menge einer Verbindung der Formel I oder II nach Anspruch 1.

27. Masse nach Anspruch 26, worin das organische Polymere ein Polyolefin ist.

28. Masse nach Anspruch 27, worin das Polyolefin Polypropylen ist.

29. Masse nach Anspruch 26, worin die Komponente (b) 1,8-Bis-(4-benzoyloxy-2,2,6,6-tetramethylpiperidin-1-oxy)octan ist.

30. Masse nach Anspruch 26, worin das organische Polymere ein Beschichtungssystem auf der Basis von Alkyd-, Acryl-, Acrylalkyd-, Polyester-, Epoxid-, Urethan-, Polyamid-, Vinyl- oder Epoxypolyesterharzen ist.

31. Masse nach Anspruch 30, dadurch **gekennzeichnet**, daß sie einen UV-Absorber oder einen zusätzlichen Lichtstabilisator enthält.

32. Verfahren zur Stabilisierung eines organischen Materials gegen oxidative, thermische oder actinische Zersetzung, dadurch **gekennzeichnet**, daß man in das organische Material eine effektive stabilisierende Menge einer Verbindung der Formel I oder II nach Anspruch 1 einarbeitet.

## Revendications

1. Composé poly(1-hydro-carbyloxy-2,2,6,6-tétraméthyl-pipéridine) de formule I ou II 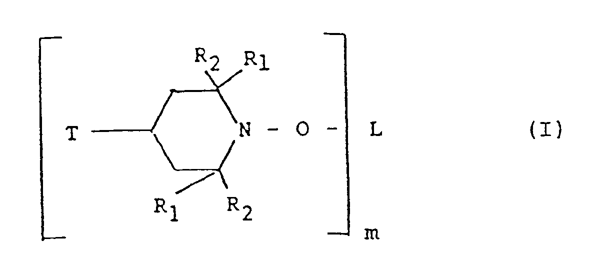 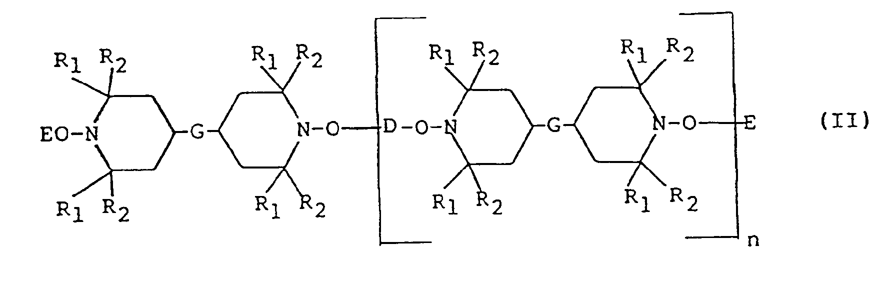 dans laquelle
m vaut de 2 à 10, n vaut de 1 à 5,
R₁ et R₂ représentent indépendamment un groupe alkyle de 1 à 4 atomes de carbone, ou R₁ et R₂ reliés ensemble représentent le groupe pentaméthylène,
L représente un radical m-valent d'un alcane de 1 à 18 atomes de carbone ou d'un alcène comportant jusqu'à 18 atomes de carbone, un radical m-valent d'un cycloalcane ou cycloalcène de 5 à 12 atomes de carbone, un radical m-valent d'un hydrocarbure bicyclique ou tricyclique saturé ou insaturé de 7 à 12 atomes de carbone ou un radical m-valent d'un groupe aryle, aryle substitué par un alkyle ou un hydrocarbure aralkyle de 6 à 15 atomes de carbone, à condition que les groupes N-O ne soient pas nécessairement liés au même atome de carbone dans L,
T est une fraction organique choisie parmi le groupe formé par R₃-COO-, R₄-OCO-, R₃-CONR₅-, 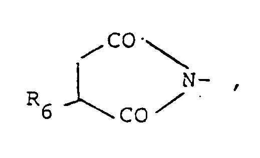 R₄NHCOCONH-, 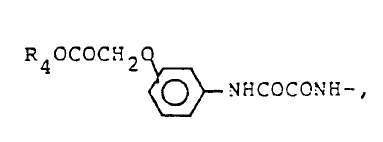 R₄OCO(CH₂)_{q}NH-, R₄OCO(CH₂)ₚCOO-, 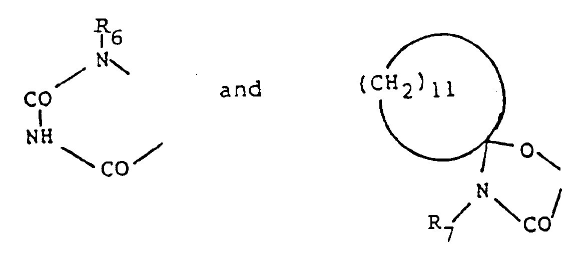 où R₃ est un radical d'un acide carboxylique aliphatique, cycloaliphatique, aliphatique insaturé ou aromatique sans le groupe carboxy,
R₄ est un radical d'un alcool aliphatique, cycloaliphatique ou aliphatique insaturé sans le groupe hydroxy,
R₅ représente un atome d'hydrogène, un groupe alkyle de 1 à 8 atomes de carbone ou 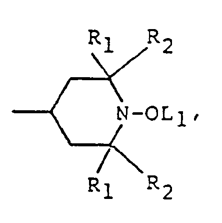 dans lequel L₁ représente un radical monovalent de la définition de L,
p vaut de 0 à 10, q vaut de 2 à 4,
R₆ représente un atome d'hydrogène ou un groupe alkyle de 1 à 18 atomes de carbone,
R₇ représente un atome d'hydrogène ou un résidu -CH₂CH₂COOC₁₂H₂₅,
D est un diradical d'un alcane ou alcène de 1 à 18 atomes de carbone, un diradical d'un cycloalcane ou d'un cycloalcène de 5 à 12 atomes de carbone, ou un diradical d'un hydrocarbure bicyclique ou tricyclique saturé ou insaturé de 7 à 12 atomes de carbone, à condition que les groupes N-O ne soient pas nécessairement liés au même atome de carbone dans D,
E est un radical monovalent d'un alcane ou d'un alcène de 1 à 18 atomes de carbone, un radical monovalent d'un cycloalcane ou cycloalcène de 5 à 12 atomes de carbone ou un radical monovalent d'un hydrocarbure bicyclique ou tricyclique saturé ou insaturé de 7 à 12 atomes de carbone,
G représente une fraction organique de liaison choisie parmi le groupe formé dans -OCO-R₈-COO-, -COO-R₉-OCO-, -NR₅-CO-R₈-CONR₅-, 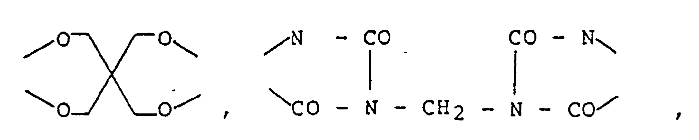 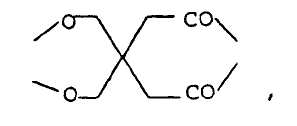 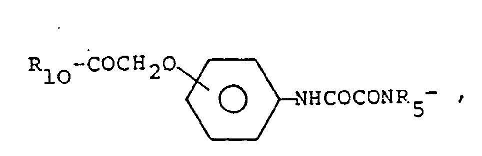 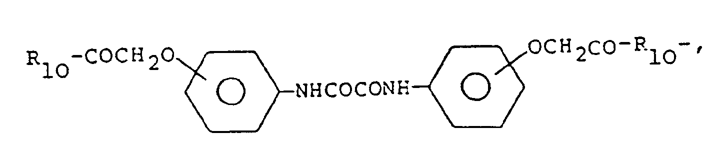 et 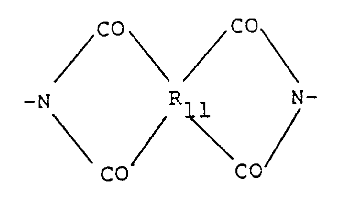 où R₈ représente une liaison directe ou un radical divalent d'un acide dicarboxylique aliphatique, cycloaliphatique, aliphatique insaturé ou aromatique sans les deux groupes carboxy,
R₉ représente un radical divalent d'un diol aliphatique, cycloaliphatique ou aliphatique insaturé sans les deux groupes hydroxyle,
R₁₀ représente -O- ou -NR₅-, et
R₁₁ représente un radical tétravalent aliphatique ou aromatique.

2. Composé de formule I selon la revendication 1, dans laquelle m vaut de 2 à 4.

3. Composé selon la revendication 2, dans lequel m vaut 2.

4. Composé de formule II selon la revendication 1, dans lequel n vaut 1 ou 2.

5. Composé de formule I ou II selon la revendication 1, dans laquelle R₁ et R₂ représente chacun un groupe méthyle.

6. Composé de formule I selon la revendication 1, dans laquelle L représente un radical m-valent de n-octane, n-heptane ou de cyclohexane.

7. Composé de formule I selon la revendication 1, dans laquelle T représente un résidu R₃-COO- où R₃ représente un groupe phényle, vinyle ou alkyle de 1 à 17 atomes de carbone.

8. Composé selon la revendication 7, dans lequel R3 représente le groupe heptadécyle.

9. Composé de formule I selon la revendication 1, dans laquelle T représente le résidu R₃-CONR₅- où R₅ rerpésente un atome d'hydrogène et R₃ représente un groupe vinyle.

10. Composé de formule I selon la revendication 1, dans laquelle T représente 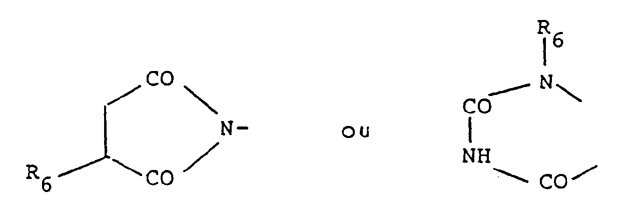 où R₆ représente un groupe dodécyle.

11. Composé de formule II selon la revendication 1 dans laquelle D représente un diradical de n-octane, de n-heptane ou de cyclohexane.

12. Composé de formule II selon la revendication 1, dans laquelle E représente un groupe octyle, heptyle ou cyclohexyle.

13. Composé de formule II selon la revendication 1, dans laquelle G représente le résidu -OCO-R₈-COO- où R₈ représente un groupe alkylène de 2 à 8 atomes de carbone ou un groupe phénylène.

14. Composé selon la revendication 13, dans lequel R₈ rerpésente un groupe éthylène ou octaméthylène.

15. Composé de formule II selon la revendication 1, dans laquelle G représente un résidu -NR₅-CO-R₈-CO-NR₅- où R₅ représente un atome d'hydrogène ou un groupe butyle et R₈ représente une liaison directe ou un groupe alkylène de 2 à 8 atomes de carbone.

16. Composé selon la revendication 15, dans lequel R₈ représente une liaison directe, un groupe éthylène ou octaméthylène.

17. Composé de formule II selon la revendication 1, dans laquelle G est

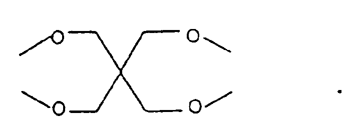

18. Composé selon la revendication 1 qui est le 1,4-bis (4-benzoyloxy-2,2,6,6-tétraméthylpipéridine-1-yloxy) -2-cyclohexène.

19. Composé selon la revendication 1 qui est le 1,8-bis-(4-benzoyloxy-2,2,6,6-tétraméthylpipéridine-1-yl-oxy)-octane.

20. Composé selon la revendication 1 qui est le bis-(4-benzoyloxy-2,2,6,6-tétraméthylpipéridine-1-yloxy)octane.

21. Composé selon la revendication 1 qui est le bis-(4-benzoyloxy-2,2,6,6-tétraméthylpipéridine-1-yloxy)méthane.

22. Composé selon la revendication 1 qui est le bis[sébacate de (1-cyclohexyloxy-2,2,6,6-tétraméthylpipéridine-4-yle) et de (1-oxy-2,2,6,6-tétraméthylpipéridine-4-yle)cyclohexanediyl..

23. Composé selon la revendication 1 qui est le bis[(sébacate de 1-heptyloxy-2,2, 6, 6-tétraméthylpipéridine-4-yle) et de (1-oxy-2,2,6,6-tétraméthylpipéridine-4-yle)] heptanediyle.

24. Composé selon la revendication 1 qui est le bis[sébacate de (1-octyloxy-2,2,6,6-tétraméthyl-pipéridine-4-yle) et de (1-oxy-2,2,6,6.tétraméthyl-pipéridine-4-yle)] octanediyle.

25. Composé selon la revendication 1 qui est le bis[sébacate de (l-octyloxy-2,2,6,6-tétraméthylpipéridine-4-yle) et de (1-oxy-2,2,6,6-tétraméthylpipéridine-4-yle)]1,8-octanediyle.

26. Composition de polymère organique stabilisé comprenant.
(a) un polymère organique sujet à une détérioration par les effets nuisibles de la lumière actinique, et
(b) une quantité stabilisatrice d'un composé de formule I ou II selon la revendication 1.

27. Composition selon la revendication 26, dans laquelle le polymère organique est une polyoléfine.

28. Composition selon la revendication 27, dans laquelle la polyoléfine est le polypropylène.

29. Composition selon la revendication 26, dans laquelle le composant (b) est le 1,8-bis(4-benzoyloxy-2,2,6,6-tétraméthylpipéridine-1-oxy)octane.

30. Composition selon la revendication 26 dans laquelle le polymère organique est un système de revêtement à base de résine alkyde, acrylique, acrylique alkyde, polyester, époxyde, uréthanne, polyamide, vinylique ou époxy-polyester.

31. Composition selon la revendication 30 qui contient un absorbant UV ou un stabilisant à la lumière supplémentaire.

32. Procédé de stabilisation d'un matériau organique contre une dégradation thermique oxydante ou actinique qui comprend l'incorporation dans le dit matériau organique d' une quantité stabilisatrice efficace d'un composé de formule I ou II selon la revendication 1.
